(19) Europäisches Patentamt
European Patent Office
Office européen des brevets

(11) Veröffentlichungsnummer: **0 433 233 B1**

(12) **EUROPÄISCHE PATENTSCHRIFT**

(45) Veröffentlichungstag der Patentschrift: **25.01.95**

(51) Int. Cl.[6]: **C07D 333/60**, A01N 43/12

(21) Anmeldenummer: **90810952.3**

(22) Anmeldetag: **05.12.90**

# (54) Heterocyclische Verbindungen.

(30) Priorität: **14.12.89 CH 4499/89**

(43) Veröffentlichungstag der Anmeldung:
**19.06.91 Patentblatt 91/25**

(45) Bekanntmachung des Hinweises auf die Patenterteilung:
**25.01.95 Patentblatt 95/04**

(84) Benannte Vertragsstaaten:
**AT BE CH DE ES FR GB IT LI LU NL**

(56) Entgegenhaltungen:
**EP-A- 0 342 459**
**US-A- 4 816 476**

(73) Patentinhaber: **CIBA-GEIGY AG**
**Klybeckstrasse 141**
**CH-4002 Basel (CH)**

(72) Erfinder: **Trah, Stephan, Dr.**
**8 Erlenweg**
**W-7800 Freiburg im Breisgau (DE)**
Erfinder: **Gantz, François**
**6, Rue des Bosquets**
**W-68170 Rixheim (FR)**

(74) Vertreter: **Roth, Bernhard M. et al**
**Patentabteilung der CIBA-Geigy AG**
**Klybeckstrasse 141**
**CH-4002 Basel (CH)**

EP 0 433 233 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft heterocyclische Verbindungen, und zwar Benzo[b]thiophen-Derivate der allgemeinen Formel

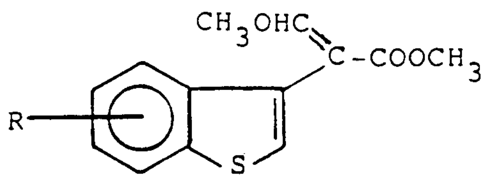

I

worin

R Halogen, $C_{1-4}$-Alkyl, $C_{1-4}$-Halogenalkyl, $C_{1-4}$-Alkoxy-$C_{1-4}$-alkyl, Aryl-$C_{1-4}$-alkoxy-$C_{1-4}$-alkyl, Aryloxy-$C_{1-4}$-alkyl, Heteroaryloxy-$C_{1-4}$-alkyl, Arylthio-$C_{1-4}$-alkyl, Heteroarylthio-$C_{1-4}$-alkyl,$C_{2-5}$-Alkanoyloxy-$C_{1-4}$-alkyl, (gegebenenfalls mit $C_{1-4}$-Alkyl substituiertes $C_{3-6}$-Cycloalkyl)carbonyloxy-$C_{1-4}$-alkyl, Aroyloxy-$C_{1-4}$-alkyl, Aryl-$C_{2-5}$-alkanoyloxy-$C_{1-4}$-alkyl, Heteroaryl-$C_{2-5}$-alkanoyloxy-$C_{1-4}$-alkyl, $C_{1-4}$-Alkoxy, Aryloxy oder eine der Gruppen (a) bis (e)

$R^1CH=CH$ (a)

$R^2R^3NCH_2$ (b)

$R^4R^5C=NOCH_2$ (c)

$R^6N=CH$ (d)

$R^7R^8N\text{-}N=CH$ (e)

$R^1$ Aryl oder Heteroaryl,

$R^2$ und $R^3$ unabhängig voneinander Wasserstoff, $C_{1-4}$-Alkyl, Aryl-$C_{1-4}$-alkyl, Aryl oder Heteroaryl

oder

$R^2$ und $R^3$ zusammen mit dem Stickstoffatom, an das sie gebunden sind, einen 5- oder 6-gliedrigen, gegebenenfalls ein Sauerstoff- oder Schwefelatom enthaltenden Ring,

$R^4$ und $R^5$ unabhängig voneinander Wasserstoff, $C_{1-4}$-Alkyl, $C_{1-4}$-Halogenalkyl, $C_{1-4}$-Alkoxy-$C_{1-4}$-alkyl, $C_{1-4}$-Alkylthio-$C_{1-4}$-alkyl, Aryl-$C_{1-4}$-alkyl, $C_{3-6}$-Cycloalkyl, Aryl, Heteroaryl, $C_{1-4}$-Alkoxy oder Aroyl,

oder

$R^4$ und $R^5$ zusammen mit dem Kohlenstoffatom, an das sie gebunden sind, einen 5- oder 6-gliedrigen, gegebenenfalls einen oder zwei ankondensierte Benzolringe aufweisenden carbocyclischen oder heterocyclischen Ring,

$R^6$ Aryl, Heteroaryl, $C_{1-4}$-Alkoxy, Aryl-$C_{1-4}$-alkoxy oder $C_{3\ \text{oder}\ 4}$-Alkenyloxy,

und

$R^7$ und $R^8$ unabhängig voneinander Wasserstoff, $C_{1-4}$-Alkyl, Aryl, Heteroaryl oder Arylsulfonyl bedeuten.

Die erfindungsgemässen Verbindungen besitzen fungizide Eigenschaften und eignen sich als fungizide Wirkstoffe, insbesondere zur Verwendung in der Landwirtschaft und im Gartenbau.

Die Erfindung betrifft ferner ein Verfahren zur Herstellung der erfindungsgemässen Verbindungen, fungizide Mittel, die solche Verbindungen als Wirkstoffe enthalten, sowie die Verwendung solcher Verbindungen und Mittel zur Bekämpfung von Fungi in der Landwirtschaft und im Gartenbau.

In der obigen Formel I können sämtliche Gruppen "Alkyl", als solche oder als Teil grösserer Gruppen, z.B. Heteroarylalkyl, je nach Anzahl deren Kohlenstoffatome geradkettig oder verzweigt sein. Ein allfällig vorhandenes Halogenatom ist Fluor, Chlor, Brom oder Jod, wobei Fluor, Chlor und Brom bevorzugt sind. Eine Halogenalkylgruppe kann einen oder mehrere Halogensubstituenten aufweisen, die gleich oder verschieden sein können. Unter Aryl ist insbesondere Phenyl oder Naphthyl, unter Heteroaryl eine

heterocyclische Gruppe mit aromatischem Charakter, wie beispielsweise Pyridyl, Furyl, Thienyl, Isoxazolyl, Thiazolyl, Imidazolyl, Pyrimidinyl oder 1,3,4-Thiadiazolyl, oder eine solche Gruppe mit ankondensiertem Benzol, z.B. Benzoxazolyl, zu verstehen. Dies gilt auch für Aryl oder Heteroaryl als Teil einer grösseren Gruppe, z.B. Aralkyl bzw. Heteroarylalkyl. Die Aryl- und Heteroarylgruppen können jeweils einen oder mehrere Substituenten aufweisen, die geeigneterweise einer oder mehrere aus Halogen, $C_{1-4}$-Alkyl, $C_{1-4}$-Halogenalkyl,$C_{1-4}$-Alkylthio-$C_{1-4}$-alkyl, $C_{1-4}$-Alkoxy, $C_{1-4}$-Halogenalkoxy, Aryl-$C_{1-4}$-alkoxy, $C_{3-6}$-Alkenyloxy, Aryloxy, $C_{1-4}$-Alkylthio, $C_{3-6}$-Cycloalkyl-$C_{1-4}$-alkylthio, Nitro und Tri($C_{1-4}$-alkyl)silyl ausgewählte Substituenten sind. Zudem kann eine Phenylgruppe einen ankondensierten Tetrahydrofuran-, Dioxolan- oder Dioxanring aufweisen, so dass die entsprechende Gruppe 2,3-Dihydrobenzofuranyl, 1,3-Benzodioxolanyl bzw. 1,4-Benzodioxanyl ist. Auch der 5- oder 6-gliedrige heterocyclische Ring $R^2R^3N$ [Teil der Gruppe (b)] kann substituiert sein, insbesondere mit einem oder mehreren $C_{1-4}$-Alkylgruppen.

Der Substituent R befindet sich jeweils in der 4-, 5-, 6- oder 7-Stellung des Benzo[b]thiophenkerns, wobei die 5-Stellung bevorzugt ist.

Falls in den Verbindungen der Formel I asymmetrische Kohlenstoffatome vorliegen, treten die Verbindungen in optisch aktiver Form auf. Allein aufgrund des Vorhandenseins der aliphatischen Doppelbindung treten die Verbindungen auf jeden Fall in der [E]- oder [Z]-Form auf. Ferner kann Atropisomerie auftreten. Die Formel I soll all diese möglichen isomeren Formen sowie deren Gemische, z.B. racemische Gemische und beliebige [E/Z]-Gemische, umfassen.

Eine besondere Untergruppe von Verbindungen der Formel I besteht aus denjenigen Verbindungen I, in denen R Halogen,$C_{1-4}$-Alkyl, $C_{1-4}$-Halogenalkyl, $C_{1-4}$-Alkoxy-$C_{1-4}$-alkyl, Aryloxy-$C_{1-4}$-alkyl, Heteroaryloxy-$C_{1-4}$-alkyl, Arylthio-$C_{1-4}$-alkyl, Heteroarylthio-$C_{1-4}$-alkyl,$C_{1-4}$-Alkoxy, Aryloxy oder eine der oben angegebenen Gruppen (a) bis (d) bedeutet, $R^1$, $R^2$ und $R^3$ die oben angegebenen Bedeutungen besitzen, und $R^4$ und $R^5$ unabhängig voneinander Wasserstoff, $C_{1-4}$-Alkyl, $C_{1-4}$-Halogenalkyl, $C_{1-4}$-Alkoxy-$C_{1-4}$-alkyl, $C_{3-6}$-Cycloalkyl, Aryl oder Heteroaryl und $R^6$ Aryl oder Heteroaryl bedeuten.

Besonders bevorzugte einzelne Verbindungen der Formel I sind:

α-{5-(α-Methyl-m-trifluormethyl-benzylidenaminooxymethyl)-3-benzo[b]thienyl}-β-methoxyacrylsäure-methylester,

α-{5-(α-[n-Propyl]-benzylidenaminooxymethyl)-3-benzo[b]thienyl}-β-methoxyacrylsäure-methylester,

α-{5-[α-(2-Pyridyl)-äthylidenaminooxymethyl]-3-benzo[b]thienyl}-β-methoxyacrylsäure-methylester,

α-{5-(α-Cyclopropyl-4-chlorbenzylidenaminooxymethyl)-3-benzo[b]thienyl}-β-methoxyacrylsäure-methylester,

α-{5-Methoxy-3-benzo[b]thienyl}-β-methoxyacrylsäure-methylester,

α-{5-Aethoxy-3-benzo[b]thienyl}-β-methoxyacrylsäure-methylester,

α-{5-[(a-Phenyläthyl)methylaminomethyl]-3-benzo[b]thienyl}-β-methoxyacrylsäure-methylester,

α-{5-(Dicyclopropylmethylidenaminooxymethyl)-3-benzo[b]thienyl}-β-methoxyacrylsäure-methylester,

α-{5-[(Cyclopropyl)(2-pyridyl)methylidenaminooxymethyl]-3-benzo[b]thienyl}-β-methoxyacrylsäure-methylester,

α-{5-(α-Cyclopropyl-m-trifluormethyl-benzylidenaminooxymethyl)-3-benzo[b]thienyl}-β-methoxyacrylsäure-methylester,

α-{5-(α-Cyclopropyl-benzylidenaminooxymethyl)-3-benzo[b]thienyl}-β-methoxyacrylsäure-methylester,

α-{5-[β-Methyl-α-(2-pyridyl)-propylidenaminooxymethyl]-3-benzo[b]thienyl)-β-methoxyacrylsäure-methylester und

α-{5-(2-Isopropyl-4-methyl-2,5-dihydro-thiazol-5-ylidenaminooxymethyl)-3-benzo[b]thienyl}-β-methoxyacrylsäure-methylester.

Das erfindungsgemässe Verfahren zur Herstellung der Verbindungen der allgemeinen Formel I ist dadurch gekennzeichnet, dass man

a) zwecks Herstellung derjenigen Verbindungen der Formel I, in denen R verschieden von einer Gruppe (b), (c), (d) oder (e) ist, einen α-(3-Benzo[b]thienyl)-β-hydroxyacrylsäure-methylester der allgemeinen Formel

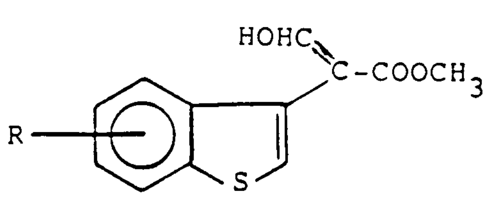

II

worin R die oben angegebene Bedeutung ausser einer Gruppe (b), (c), (d) oder (e) besitzt, mit einem Methylierungsmittel behandelt,

b) zwecks Herstellung derjenigen Verbindungen der Formel I, in denen R Brommethyl bedeutet, einen α-(Methyl-3-benzo[b]thienyl)-β-methoxyacrylsäure-methylester der allgemeinen Formel

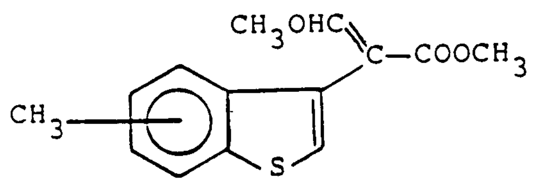

Ia

mit N-Bromsuccinimid behandelt,

c) zwecks Herstellung derjenigen Verbindungen der Formel I, in denen R $C_{1-4}$-Alkoxy-methyl, Aryl-$C_{1-4}$-alkoxy-methyl, Aryloxymethyl, Heteroaryloxymethyl, Arylthiomethyl, Heteroarylthiomethyl, $C_{2-5}$-Alkanoyloxy-methyl, (gegebenenfalls mit $C_{1-4}$-Alkyl substituiertes $C_{3-6}$-Cycloalkyl)carbonyloxymethyl, Aroyloxymethyl, Aryl-$C_{2-5}$ -alkanoyloxy-methyl, Heteroaryl-$C_{2-5}$ -alkanoyl-oxy-methyl, eine Gruppe (b) oder eine Gruppe (c) bedeutet, einen α-(Brommethyl-3-benzo[b]thienyl)-β-methoxyacrylsäure-methylester der allgemeinen Formel

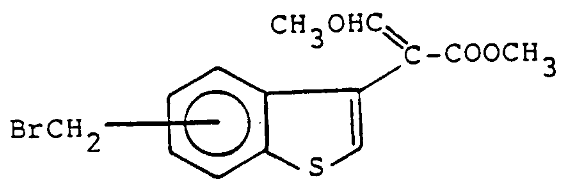

Ib

mit einer Hydroxyverbindung, einer Mercaptoverbindung, einer Carbonsäure, einem Amin bzw. einem Oxim der allgemeinen Formel

R'-H III

worin R′ $C_{1-4}$-Alkoxy, Aryl-$C_{1-4}$-alkoxy, Aryloxy, Heteroaryloxy, $C_{2-5}$-Alkanoyloxy, (gegebenenfalls mit $C_{1-4}$-Alkyl substituiertes $C_{3-6}$-Cycloalkyl)-carbonyloxy, Aroyloxy, Aryl-$C_{2-5}$ -alkanoyloxy, Heteroaryl-$C_{2-5}$-alkanoyloxy, Arylthio, Heteroarylthio, eine Gruppe $R^2R^3N$ (b′) oder eine Gruppe $R^4R^5C=NO$ (c′) bedeutet,

umsetzt,

d) zwecks Herstellung derjenigen Verbindungen der Formel I, in denen R eine Gruppe (a) bedeutet, einen {3-(α-Methoxycarbonyl-β-methoxyvinyl)-benzo[b]thienyl}methylphosphonsäure-dialkylester der allgemeinen Formel

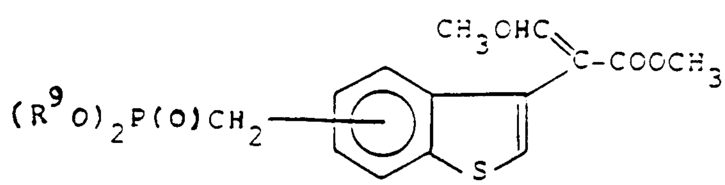

IV

worin $R^9$ $C_{1-4}$-Alkyl bedeutet, mit einem Aldehyd der allgemeinen Formel

$R^1CHO$ V

worin $R^1$ die oben angegebene Bedeutung besitzt, umsetzt, oder

e) zwecks Herstellung derjenigen Verbindungen der Formel I, in denen R eine Gruppe (d) oder eine Gruppe (e) bedeutet, einen α-(Formyl-3-benzo[b]thienyl)-β-methoxyacrylsäure-methylester der allgemei-

nen Formel

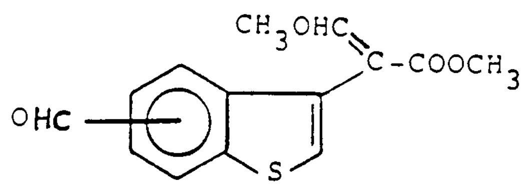

VI

mit einem Amin, Hydroxylamin bzw. Hydrazin der allgemeinen Formel

$R^6NH_2$ VII

bzw.

$R^7R^8N\text{-}NH_2$ VIII

worin $R^6$, $R^7$ und $R^8$ die oben angegebenen Bedeutungen besitzen,

umsetzt.

Bei der Verfahrensvariante a) handelt es sich um die Methylierung des β-Hydroxysubstituenten des Acrylsäurederivats II. Diese Methylierung kann unter den diesbezüglich üblichen Reaktionsbedingungen durchgeführt werden. So erfolgt die Umsetzung zweckmässigerweise in einem inerten organischen Lösungsmittel, wie Aceton, Dimethylformamid oder Dimethylsulfoxid, in Gegenwart einer Base, wie Natriumhydrid oder eines Alkalimetallcarbonats, z.B. Natrium- oder Kaliumcarbonat, bei Temperaturen zwischen 20 °C und 80 °C, vorzugsweise im Temperaturbereich von 40 °C bis 60 °C.

Auch die Bromierung nach Verfahrensvariante b) kann auf an sich bekannte Weise durchgeführt werden, und zwar zweckmässigerweise in einem inerten organischen Lösungsmittel, wie einem halogenierten Kohlenwasserstoff, z.B. Tetrachlorkohlenstoff, in Gegenwart eines Radikale bildenden Initiators, z.B. Azoisobutyronitril oder Dibenzoylperoxid, bei Tempera- turen zwischen 70 °C und 90 °C, vorzugsweise bei ca. 80 °C. Ein Beispiel einer solchen Bromierung ist von Horner et al. in Angew. Chem. 71, 349-365 (1959) beschrieben.

Die Umsetzung nach Verfahrensvariante c) stellt eine nucleophile Substitution dar, die zweckmässigerweise in einem inerten organischen Lösungsmittel, wie einem aliphatischen oder cyclischen Aether, z.B. Dimethoxyäthan, Tetrahydrofuran oder Dioxan; einem aliphatischen Keton, z.B. Aceton; Dimethylformamid oder Dimethylsulfoxid, in Gegenwart einer Base, wie Natriumhydrid, eines Alkalimetallcarbonats, z.B. Natrium- oder Kaliumcarbonat, eines tertiären Amins, z.B. eines Trialkylamins, insbesondere Diazabicyclononan oder Diazabicycloundecan, oder Silberoxid, bei Temperaturen zwischen 0 °C und 100 °C, vorzugsweise im Temperaturbereich von 20 °C bis 80 °C, erfolgt.

Bei der Umsetzung nach Verfahrensvariante d) handelt es sich um eine Wittig-Horner-Reaktion, die zweckmässigerweise in einem inerten organischen Lösungsmittel, wie einem aliphatischen oder cyclischen Aether, z.B. 1,2-Dimethoxyäthan oder Tetrahydrofuran, in Gegenwart einer Base, z.B. Natriumhydrid, bei Temperaturen zwischen 20 °C und 80 °C durchgeführt wird. Beispiele einer solchen Reaktion sind in J.A.C.S. 83, 1733-1738 (1961) (Wadsworth et al.) zu finden.

Die Kondensation nach Verfahrensvariante e) kann auf an sich bekannte Weise durchgeführt werden, und zwar zweckmässigerweise in einem organischen Lösungsmittel, wie einem Alkanol, z.B. Methanol oder Aethanol, Tetrahydrofuran oder Pyridin, gegebenenfalls unter Zusatz einer katalytischen Menge p-Toluolsulfonsäure, bei Temperaturen zwischen 20 °C und 110 °C.

Die Isolierung und Reinigung der so hergestellten Verbindungen der Formel I kann nach an sich bekannten Methoden erfolgen. Ebenfalls nach an sich bekannten Methoden können allfällig erhaltene Isomerengemische, z.B. E/Z-Isomerengemische, in die reinen Isomeren aufgetrennt werden, beispielsweise durch Chromatographie oder fraktionierte Kristallisation.

Die als Ausgangsmaterialien in der Verfahrensvariante a) verwendeten α-(3-Benzo[b]thienyl)-β-hydroxyacrylsäure-methylester der Formel II können in an sich bekannter Weise, z.B. gemäss dem nachfolgenden Reaktionsschema, hergestellt werden:

Reaktionsschema

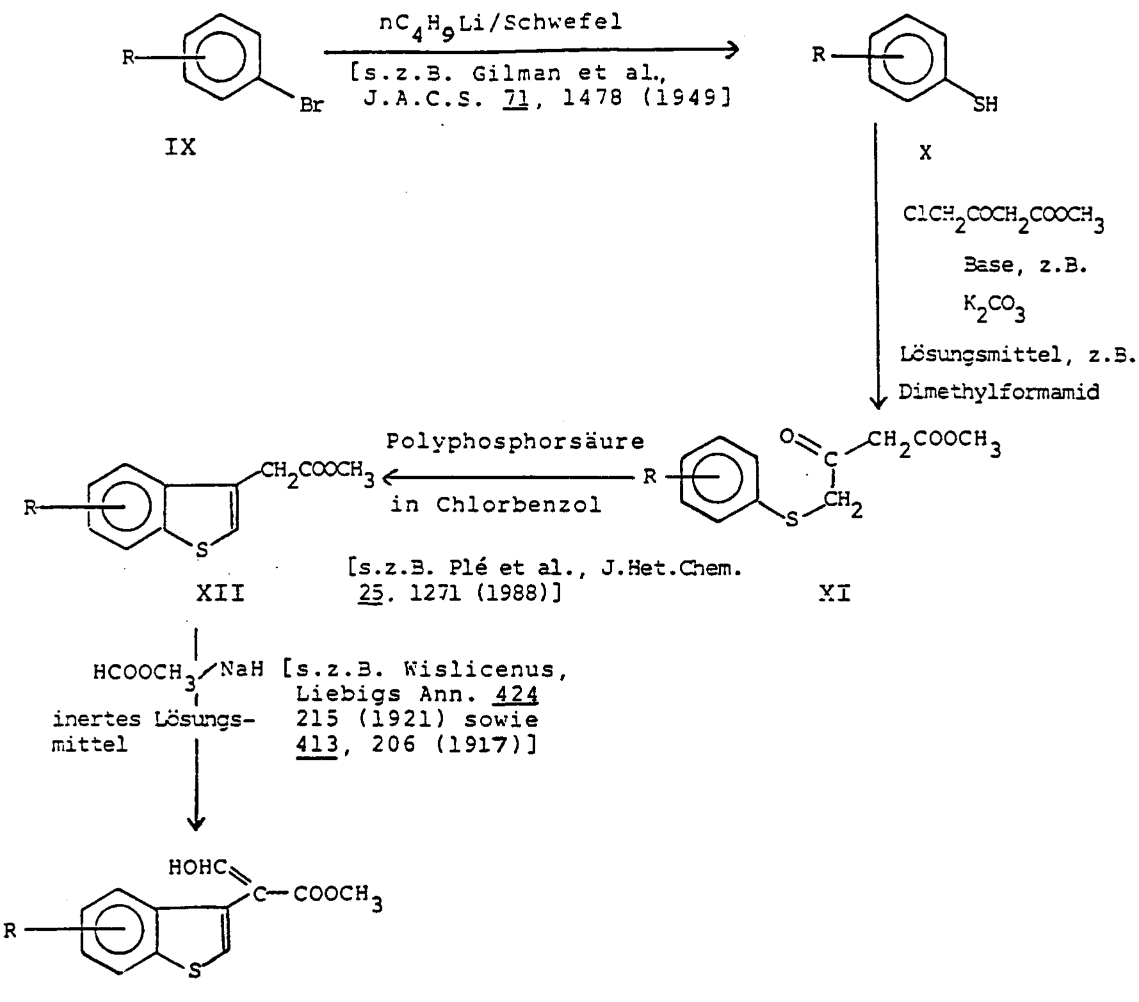


IX X XI XII II

Die als Ausgangsmaterialien in der Verfahrensvariante d) verwendeten {3-(α-Methoxycarbonyl-β-methoxyvinyl)-benzo[b]thienyl}methylphosphonsäure-dialkylester der Formel IV können auf an sich bekannte Weise durch Umsetzung des entsprechenden α-(Brommethyl-3-benzo[b]thienyl)-β-methoxyacrylsäure-methylesters der oben angegebenen Formel Ib mit einem Trialkylester von phosphoriger Säure hergestellt werden [siehe z.B. Houben-Weyl, Methoden der organischen Chemie, Bd. 12/I, 433ff (1963)].

Auch die als Ausgangsmaterialien in der Verfahrensvariante e) verwendeten α-(Formyl-3-benzo[b]-thienyl)-β-methoxyacrylsäure-methylester der Formel VI können auf an sich bekannte Weise hergestellt werden, und zwar durch Umsetzung des entsprechenden α-(Brommethyl-3-benzo[b]thienyl)-β-methoxyacrylsäure-methylesters der oben angegebenen Formel Ib mit Natriumhydrogencarbonat und Dimethylsulfoxid [die Kornblum-Reaktion, siehe z.B. J.A.C.S. 79, 6562ff (1957)].

Bei den als Ausgangsmaterialien in der Verfahrensvariante b) verwendeten α-(Methyl-3-benzo[b]thienyl)-β-methoxyacrylsäure-methylestern der Formel Ia sowie in der Verfahrensvariante c) verwendeten α-(Brommethyl-3-benzo[b]thienyl)-β-methoxyacrylsäure-methylestern der Formel Ib handelt es sich um Untergruppen der Verbindungen der Formel I, die beispielsweise nach Verfahrensvariante a) bzw. b) hergestellt werden können. Die Ausgangsmaterialien der Formeln III, V, VII, VIII und IX sind entweder bekannt oder können nach an sich bekannten Methoden hergestellt werden.

Die erfindungsgemässen Verbindungen besitzen fungizide Wirkung und können dementsprechend zur Bekämpfung von Pilzen in der Landwirtschaft, im Gartenbau sowie in der Holzverarbeitung Verwendung

finden. Sie eignen sich insbesondere zur Hemmung des Wachstums oder zur Vernichtung von phytopathogenen Pilzen auf Pflanzenteilen, z.B. Blättern, Stengeln, Wurzeln, Knollen, Früchten oder Blüten, und auf Saatgut sowie von im Erdboden auftretenden Schadpilzen. Ferner können mit den erfindungsgemässen Verbindungen holzabbauende und holzverfärbende Pilze bekämpft werden. Die erfindungsgemässen Verbindungen sind beispielsweise wirksam bei der Bekämpfung von Pilzen der Klassen Deuteromycetes, Ascomycetes, Basidiomycetes und Phycomycetes.

Besonders eignen sich die erfindungsgemässen Verbindungen zur Bekämpfung der folgenden Schaderreger:

Echte Mehltaupilze (z.B. Erysiphe graminis, Erysiphe cichoracearum, Podosphaera leucotricha, Uncinula necator, Sphaerotheca spp.)

Rostpilze (z.B. Puccinia tritici, Puccinia recondita, Puccinia hordei, Puccinia coronata, Puccinia striiformis, Puccinia arachidis, Hemileia vastatrix, Uromyces fabae)

Schorfpilze (z.B. Venturia inaequalis)

Cercospora spp. (z.B. Cercospora arachidicola, Cercospora beticola)

Mycosphaerella spp. (z.B. Mycosphaerella fijiensis)

Alternaria spp. (z.B. Alternaria brassicae, Alternaria mali)

Septoria spp. (z.B. Septoria nodorum)

Heminthosporium spp. (z.B. Helminthosporium teres, Helminthosporium oryzae)

Plasmopara spp. (z.B. Plasmopara viticola)

Pseudoperonospora spp. (z.B. Pseudoperonospora cubensis)

Phytophtora spp. (z.B. Phytophtora infestans)

Pseudocercosporella spp. (z.B. Pseudocercosporella herpotrichoides)

Pyricularia spp. (z.B. Pyricularia oryzae)

Ferner wirken die Verbindungen beispielsweise gegen Pilze der Gattungen Tilletia, Ustilago, Rhizoctonia, Verticillium, Fusarium, Pythium, Gaeumannomyces, Sclerotinia, Monilia, Botrytis, Peronospora, Bremia, Gloeosporium, Cercosporidium, Penicillium, Ceratocystis, Rhynchosporium, Pyrenophora, Diaporthe, Ramularia und Leptosphaeria. Gewisse Vertreter der erfindungsgemässen Verbindungen besitzen zudem Wirkung gegen holzschädigende Pilze, wie beispielsweise der Gattungen Coniophora, Gloeophyllum, Poria, Merulius, Trametes, Aureobasidium, Sclerophoma und Trichoderma.

Die erfindungsgemässen Verbindungen zeichnen sich durch prophylaktische und kurative Wirkung aus.

Die erfindungsgemässen Verbindungen wirken gegen phytopathogene Pilze unter Gewächshausbedingungen bereits bei Konzentrationen von 0,5 mg bis 500 mg Wirkstoff pro Liter Spritzbrühe. Im Freiland werden vorteilhaft Dosierungen von 20 g bis 1 kg Wirkstoff der Formel I pro Hektar und Behandlung zur Anwendung gebracht. Zur Bekämpfung von samen- oder bodenbürtigen Pilzen im Beizverfahren werden mit Vorteil Dosierungen von 0,01 g bis 1,0 g Wirkstoff der Formel I pro kg Samen verwendet.

Die erfindungsgemässen Verbindungen können zu verschiedenartigen Mitteln, z.B. Lösungen, Suspensionen, Emulsionen, emulgierbaren Konzentraten und pulverförmigen Präparaten, formuliert werden. Die erfindungsgemässen fungiziden Mittel sind dadurch gekennzeichnet, dass sie eine wirksame Menge mindestens einer Verbindung der allgemeinen Formel I, wie oben definiert, sowie Formulierungshilfsstoffe enthalten. Die Mittel enthalten zweckmässigerweise zumindest einen der folgenden Formulierungshilfsstoffe:

Feste Trägerstoffe; Lösungs- bzw. Dispersionsmittel; Tenside (Netzmittel und Emulgatoren); Dispergiermittel (ohne Tensidwirkung); und Stabilisatoren.

Als feste Trägerstoffe kommen im wesentlichen in Frage: natürliche Mineralstoffe, wie Kaolin, Tonerden, Kieselgur, Talkum, Bentonit, Kreide, z.B. Schlämmkreide, Magnesiumcarbonat, Kalkstein, Quarz, Dolomit, Attapulgit, Montmorillonit und Diatomeenerde; synthetische Mineralstoffe, wie hochdisperse Kieselsäure, Aluminiumoxid und Silikate; organische Stoffe, wie Cellulose, Stärke, Harnstoff und Kunstharze; und Düngemittel, wie Phosphate und Nitrate, wobei solche Trägerstoffe z.B. als Granulate oder Pulver vorliegen können.

Als Lösungs- bzw. Dispersionsmittel kommen im wesentlichen in Frage: Aromaten, wie Toluol, Xylole, Benzol und Alkylnaphthaline; chlorierte Aromaten und chlorierte aliphatische Kohlenwasserstoffe, wie Chlorbenzole, Chloräthylene und Methylenchlorid; aliphatische Kohlenwasserstoffe, wie Cyclohexan und Paraffine, z.B. Erdölfraktionen; Alkohole, wie Butanol und Glykol, sowie deren Aether und Ester; Ketone, wie Aceton, Methyläthylketon, Methylisobutylketon und Cyclohexanon; und stark polare Lösungs- bzw. Dispersionsmittel, wie Dimethylformamid, N-Methylpyrrolidon und Dimethylsulfoxid, wobei solche Lösungs- bzw. Dispersionsmittel vorzugsweise Flammpunkte von mindestens 30°C und Siedepunkte von mindestens 50°C aufweisen, und Wasser. Unter den Lösungs- bzw. Dispersionsmitteln kommen auch in Frage sogenannte verflüssigte gasförmige Streckmittel oder Trägerstoffe, die solche Produkte sind, welche bei Raumtemperatur und unter Normaldruck gasförmig sind. Beispiele solcher Produkte sind insbesondere

Aerosol-Treibgase, wie Halogenkohlenwasserstoffe, z.B. Dichlordifluormethan. Im Falle der Benutzung von Wasser als Lösungsmittel können z.B. auch organische Lösungsmittel als Hilfslösungsmittel verwendet werden.

Die Tenside (Netzmittel und Emulgatoren) können nicht-ionische Verbindungen sein, wie Kondensationsprodukte von Fettsäuren, Fettalkoholen oder fettsubstituierten Phenolen mit Aethylenoxid; Fettsäureester und -äther von Zuckern oder mehrwertigen Alkoholen; die Produkte, die aus Zuckern oder mehrwertigen Alkoholen durch Kondensation mit Aethylenoxid erhalten werden; Blockpolymere von Aethylenoxid und Propylenoxid; oder Alkyldimethylaminoxide.

Die Tenside können auch anionische Verbindungen darstellen, wie Seifen; Fettsulfatester, z.B. Dodecylnatriumsulfat, Octadecylnatriumsulfat und Cetylnatriumsulfat; Alkylsulfonate, Arylsulfonate und fettaromatische Sulfonate, wie Alkylbenzolsulfonate, z.B. Calcium-dodecylbenzolsulfonat, und Butylnaphthalinsulfonate; und komplexere Fettsulfonate, z.B. die Amidkondensationsprodukte von Oelsäure und N-Methyltaurin und das Natriumsulfonat von Dioctylsuccinat.

Die Tenside können schliesslich kationische Verbindungen sein, wie Alkyldimethylbenzylammoniumchloride, Dialkyldimethylammoniumchloride, Alkyltrimethylammoniumchloride und äthoxylierte quaternäre Ammoniumchloride.

Als Dispergiermittel (ohne Tensidwirkung) kommen im wesentlichen in Frage: Lignin, Natrium- und Ammoniumsalze von Ligninsulfonsäure, Natriumsalze von Maleinsäureanhydrid-Diisobutylen-Copolymeren, Natrium- und Ammoniumsalze von sulfonierten Polykondensationsprodukten aus Naphthalin und Formaldehyd, und Sulfitablaugen.

Als Dispergiermittel, die sich insbesondere als Verdickungs-bzw. Antiabsetzmittel eignen, können z.B. Methylcellulose, Carboxymethylcellulose, Hydroxyäthylcellulose, Polyvinylalkohol, Alginate, Caseinate und Blutalbumin eingesetzt werden.

Beispiele von geeigneten Stabilisatoren sind säurebindende Mittel, z.B. Epichlorhydrin, Phenylglycidäther und Soyaepoxide; Antioxidantien, z.B. Gallussäurester und Butylhydroxytoluol; UV-Absorber, z.B. substituierte Benzophenone, Diphenylacrylnitrilsäureester und Zimtsäureester; und Deaktivatoren, z.B. Salze der Aethylendiamintetraessigsäure und Polyglykole.

Die erfindungsgemässen fungiziden Mittel können neben den Wirkstoffen der Formel I auch andere Wirkstoffe enthalten, z.B. anderweitige fungizide Mittel, insektizide und akarizide Mittel, Bakterizide, Pflanzenwachstumsregulatoren und Düngemittel. Solche Kombinationsmittel eignen sich zur Verbreiterung des Wirkungsspektrums oder zur spezifischen Beeinflussung des Pflanzenwachstums.

Im allgemeinen enthalten die erfindungsgemässen fungiziden Mittel, je nach deren Art, zwischen 0,0001 und 85 Gewichtsprozent an erfindungsgemässer Verbindung bzw. erfindungsgemässen Verbindungen als Wirkstoff(en). Sie können in einer Form vorliegen, die sich für die Lagerung und den Transport eignet. In solchen Formen, z.B. emulgierbaren Konzentraten, ist die Wirkstoffkonzentration normalerweise im höheren Bereich des obigen Konzentrationsintervalls. Diese Formen können dann mit gleichen oder verschiedenen Formulierungshilfsstoffen bis zu Wirkstoffkonzentrationen verdünnt werden, die sich für den praktischen Gebrauch eignen, und solche Konzentrationen liegen normalerweise im niedrigeren Bereich des obigen Konzentrationsintervalls. Emulgierbare Konzentrate enthalten im allgemeinen 5 bis 85 Gewichtsprozent, vorzugsweise 25 bis 75 Gewichtsprozent, der erfindungsgemässen Verbindung(en). Als Anwendungsformen kommen u.a. gebrauchsfertige Lösungen, Emulsionen und Suspensionen, die sich beispielsweise als Spritzbrühen eignen, in Frage. In solchen Spritzbrühen können z.B. Konzentrationen zwischen 0,0001 und 20 Gewichtsprozent vorliegen. Im Ultra-Low-Volume-Verfahren können Spritzbrühen formuliert werden, in denen die Wirkstoffkonzentration vorzugsweise von 0,5 bis 20 Gewichtsprozent beträgt, während die im Low-Volume-Verfahren und im High-Volume-Verfahren formulierten Spritzbrühen vorzugsweise eine Wirkstoffkonzentration von 0,02 bis 1,0 bzw. 0,002 bis 0,1 Gewichtsprozent aufweisen.

Die erfindungsgemässen fungiziden Mittel können dadurch hergestellt werden, dass man mindestens eine erfindungsgemässe Verbindung mit Formulierungshilfsstoffen vermischt.

Die Herstellung der Mittel kann in bekannter Weise durchgeführt werden, z.B. durch Vermischen der Wirkstoffes mit festen Trägerstoffen, durch Auflösen oder Suspendieren in geeigneten Lösungs- bzw. Dispersionsmitteln, eventuell unter Verwendung von Tensiden als Netzmitteln oder Emulgatoren oder von Dispergiermitteln, durch Verdünnen bereits vorbereiteter emulgierbarer Konzentrate mit Lösungs- bzw. Dispersionsmitteln, usw.

Im Falle von pulverförmigen Mitteln kann der Wirkstoff mit einem festen Trägerstoff vermischt werden, z.B. durch Zusammenmahlen; oder man kann den festen Trägerstoff mit einer Lösung oder Suspension des Wirkstoffs imprägnieren und dann das Lösungs- bzw. Dispersionsmittel durch Abdunsten, Erhitzen oder durch Absaugen unter vermindertem Druck entfernen. Durch Zusatz von Tensiden bzw. Dispergiermitteln kann man solche pulverförmige Mittel mit Wasser leicht benetzbar machen, so dass sie in wässrige

Suspensionen, die sich z.B. als Spritzmittel eignen, übergeführt werden können.

Die erfindungsgemässen Verbindungen können auch mit einem Tensid und einem festen Trägerstoff zur Bildung eines netzbaren Pulvers vermischt werden, welches in Wasser dispergierbar ist, oder sie können mit einem festen vorgranulierten Trägerstoff zur Bildung eines granulatförmigen Produktes vermischt werden.

Wenn gewünscht, kann eine erfindungsgemässe Verbindung in einem mit Wasser nicht mischbaren Lösungsmittel, wie beispielsweise einem alicyclischen Keton, gelöst werden, das zweckmässigerweise gelösten Emulgator enthält, so dass die Lösung bei Zugabe zu Wasser selbstemulgierend wirkt. Andernfalls kann der Wirkstoff mit einem Emulgator vermischt und das Gemisch dann mit Wasser auf die gewünschte Konzentration verdünnt werden. Zudem kann der Wirkstoff in einem Lösungsmittel gelöst und danach mit einem Emulgator gemischt werden. Ein solches Gemisch kann ebenfalls mit Wasser auf die gewünschte Konzentration verdünnt werden. Auf diese Weise erhält man emulgierbare Konzentrate bzw. gebrauchsfertige Emulsionen.

Die Verwendung der erfindungsgemässen Mittel kann nach den im Pflanzenschutz bzw. in der Landwirtschaft üblichen Applikationsmethoden erfolgen. Das erfindungsgemässe Verfahren zur Bekämpfung von Fungi ist dadurch gekennzeichnet, dass man das zu schützende Gut, z.B. Pflanzen, Pflanzenteile bzw. Samen, mit einer wirksamen Menge einer erfindungsgemässen Verbindung bzw. eines erfindungsgemässen Mittels behandelt.

Die nachstehenden Beispiele illustrieren die Erfindung.

I. Herstellung der Wirkstoffe der Formel I:

Beispiel 1

Eine Lösung von 79,7 g 5-Methyl-3-benzo[b]thienylessigsäure-methylesterin 200 ml Dimethylformamid und 200 ml Ameisensäuremethylester wird langsam einer Suspension von 10,4 g Natriumhydrid in 100 ml Dimethylformamid zugetropft. Nach 2-stündigem Rühren des Reaktionsgemisches bei Raumtemperatur wird es mit verdünnter Salzsäure angesäuert und danach mit Aethylacetat extrahiert. Man wäscht die organische Phase mit Wasser, trocknet sie über wasserfreiem Natriumsulfat und engt sie unter vermindertem Druck ein. Auf diese Weise erhält man als Rohprodukt den $\alpha$-(5-Methyl-3-benzo[b]thienyl)-$\beta$-hydroxyacrylsäure-methylester, der ungereinigt als Ausgangsmaterial in der nächsten Verfahrensstufe dient.

Das obige Ausgangsmaterial wird zusammen mit 36 ml Dimethylsulfat, 69 g Kaliumcarbonat und 315 ml Aceton 2 Stunden auf Rückflusstemperatur erhitzt. Man filtriert das auf Raumtemperatur gekühlte Gemisch, engt das Filtrat unter vermindertem Druck ein und erhält somit ein gelbes Oel. Dies wird an Kieselgel mit Diäthyläther/n-Hexan (1: 1) chromatographisch gereinigt. Nach Umkristallisieren des Rohproduktes aus Methylenchlorid/n-Hexan erhält man den $\alpha$-(5-Methyl-3-benzo[b]thienyl)-$\beta$-methoxyacrylsäure-methylester als gelbe Kristalle, Smp. 114-115 °C.

Beispiele 2-10

Analog dem in Beispiel 1 beschriebenen Verfahren wird der jeweilige substituierte 3-Benzo[b]-thienylessigsäure-methylester mit Ameisensäuremethylester zum entsprechenden a-(3-Benzo[b]thienyl)-$\beta$-hydroxyacrylsäure-methylester umgesetzt und letzteres anschliessend mit Dimethylsulfat methyliert, um die in der nachstehenden Tabelle 1 aufgeführten Verbindungen der Formel I herzustellen:

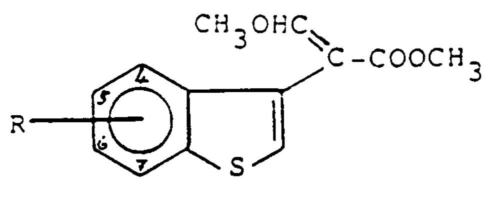

Tabelle 1

| Beispiel | R | Physikalische Daten |
|---|---|---|
| 2 | 7-Methyl | Smp. 89-90 ° C |
| 3 | 5-tert.Butyl | [E]-isomeres: Oel<br>[Z]-Isomeres: Oel |
| 4 | 5-Methoxy | Smp. 112-113 ° C |
| 5 | 7-Methoxy | Smp. 115-116 ° C |
| 6 | 6-Methoxy | Smp. 127-128 ° C |
| 7 | 5-Brom | Smp. 136-137 ° C |
| 8 | 5-Chlor | Smp. 119 ° C |
| 9 | 5-Aethoxy | Smp. 130 ° C |
| 10 | 5-Phenoxy | Oel |

Beispiel 11

Ein Gemisch von 71 g α-(5-Methyl-3-benzo[b]thienyl) -β-methoxyacrylsäure-methylester (siehe Beispiel 1), 57,8 g N-Bromsuccinimid, 1,1 g Azodiisobutyronitril und 720 ml Tetrachlorkohlenstoff wird 75 Minuten auf Rückflusstemperatur erhitzt. Nach Filtration des auf Raumtemperatur gekühlten Gemisches wird das Filtrat unter vermindertem Druck eingeengt und das verbleibende gelbe Oel an Kieselgel mit Diäthyläther/n-Hexan (1:1) chromatographisch gereinigt. Man kristallisiert das Rohprodukt aus Methylenchlorid/n-Hexan und erhält auf diese Weise den α-(5-Brommethyl-3-benzo[b]thienyl)-β-methoxyacrylsäure-methylester als hellgelbe Kristalle, Smp. 124-125 ° C.

Beispiel 12

Analog dem in Beispiel 11 beschriebenen Verfahren wird α-(7-Methyl-3-benzo[b]thienyl)-β-methoxyacrylsäure-methylester (siehe Beispiel 2) mit N-Bromsuccinimid zum α-(7-Brommethyl-3-benzo[b]-thienyl)-β-methoxyacrylsäure-methylester, Smp. 97 ° C, bromiert.

Beispiel 13

Ein Gemisch von 1 g α-(5-Brommethyl-3-benzo[b]thienyl)-β-methoxyacrylsäure-methylester (siehe Beispiel 11), 0,28 g Phenol und 2 g Kaliumcarbonat in 20 ml Dimethylformamid wird 2 Stunden bei 50 ° C erhitzt. Nach Filtration des auf Raumtemperatur gekühlten Gemisches wird das Filtrat unter vermindertem Druck eingeengt und das Rohprodukt an Kieselgel mit Diäthyläther/n-Hexan (1: 1) chromatographisch gereinigt. Man erhält nach Umkristallisieren aus Methylenchlorid/n-Hexan den α-(5-Phenoxymethyl-3-benzo-[b]thienyl)-β-methoxyacrylsäure-methylester als farblose Kristalle, Smp. 130 ° C.

Beispiele 14-55

Analog dem in Beispiel 13 beschriebenen Verfahren wird α-(5-Brommethyl-3-benzo[b]thienyl)-β-methoxyacrylsäure-methylester mit der jeweiligen Hydroxy- oder Mercaptoverbindung, der jeweiligen Carbonsäure oder dem jeweiligen Amin der Formel III umgesetzt, um die in der nachstehenden Tabelle 2 aufgeführten Verbindungen der Formel I herzustellen:

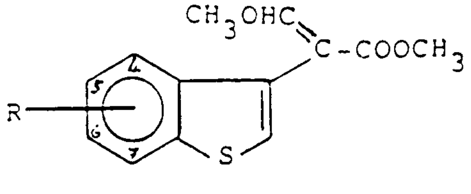

Tabelle 2

| Beispiel | R | Physikalische Daten |
|---|---|---|
| 14 | 5-[(5-Methyl-3-isoxazolyl)-oxymethyl] | Smp. 127°C |
| 15 | 5-(3-Nitrophenoxymethyl) | Smp. 128°C |
| 16 | 5-(3-Fluorphenoxymethyl) | Smp. 109-110°C |
| 17 | 5-(α,α,α-Trifluor-m-tolyloxymethyl) | Smp. 83°C |
| 18 | 5-(3-Chlorphenoxymethyl) | Smp. 111-112°C |
| 19 | 5-(4-Chlorphenoxymethyl) | Smp. 141°C |
| 20 | 5-(m-Tolyloxymethyl) | Smp. 131-132°C |
| 21 | 5-(p-Tolyloxymethyl) | Smp. 142-143°C |
| 22 | 5-(3-Aethylphenoxymethyl) | Smp. 82°C |
| 23 | 5-(4-Aethylphenoxymethyl) | Smp. 109-110°C |
| 24 | 5-(4-tert.Butyl-phenoxy-methyl) | Smp. 124-125°C |
| 25 | 5-(4-Nitro-m-tolyloxymethyl) | Smp. 140-141°C |
| 26 | 5-(4-Chlor-m-tolyloxymethyl) | Smp. 118-119°C |
| 27 | 5-Phenylthiomethyl | Smp. 95-96°C |
| 28 | 5-[5-(Cyclopropylmethyl-thio)-1,3,4-thiadiazol-2-yl-thiomethyl] | Oel |
| 29 | 5-(4-Chlorphenylthiomethyl) | Smp. 133°C |
| 30 | 5-(p-Tolylthiomethyl) | Smp. 125°C |
| 31 | 5-(4-tert.Butyl-phenylthio-methyl) | Smp. 131-132°C |
| 32 | 5-(2-Benzoxazolylthiomethyl) | Smp. 117-118°C |
| 33 | 5-Piperidinomethyl | Smp. 105-106°C |
| 34 | 5-(2,6-Dimethylmorpholino-methyl) | Oel |

| | | |
|---|---|---|
| 35 | 5-[(α-Phenyläthyl)methyl-aminomethyl] | Oel |
| 36 | 5-[(5-Methyl-3-isoxazolyl)-methylaminomethyl] | Oel |
| 37 | 5-[(1-Naphthylmethyl)methyl-aminomethyl] | Oel |
| 38 | 5-(3,4-Dimethylphenoxy-methyl) | Smp. 134-135°C |
| 39 | 5-Anilinomethyl | Smp. 141-142°C |
| 40 | 5-(α,α,α-Trifluor-m-tolylaminomethyl) | Oel |
| 41 | 5-(3-Bromphenoxymethyl) | Smp. 114-115°C |
| 42 | 5-[(m-Tolyl)methylamino-methyl] | Oel |
| 43 | 5-(8-Chinolinyloxymethyl) | Oel |
| 44 | 5-Methoxymethyl | Oel |
| 45 | 5-(α-Methylbenzyloxy-methyl) | Oel |
| 46 | 5-[(1-Methylcyclopropyl) carbonyloxymethyl | Oel |
| 47 | 5-(Benzylmethylaminomethyl) | Oel |
| 48 | 5-(4,6-Dimethylpyrimidin-2-ylthiomethyl) | Smp. 118-120°C |
| 49 | 5-(3-Pyridylacetoxymethyl) | Smp. 77-78°C |
| 50 | 5-(α,α,α-Trifluor-p-tolylacetoxymethyl) | Smp. 105-106°C |
| 51 | 5-(2,4-Dichlorbenzoyloxy methyl) | Smp. 119°C |
| 52 | 5-Cyclopropylcarbonyloxy-methyl | Oel |
| 53 | 5-(2,4-Dichlorphenyl-acetoxymethyl) | Smp. 99-100°C |
| 54 | 5-(3-Benzo[b]thienyl-acetoxymethyl | Oel |
| 55 | 5-(3,4-Dichlorphenyl-acetoxymethyl) | Oel |

Beispiel 56

Zu 77 mg Natriumhydrid in 20 ml Dimethylformamid gibt man 1 g α-(5-Brommethyl-3-benzo[b]thienyl) -β-methoxyacrylsäure-methylester (siehe Beispiel 11) und 0,59 g 3-Trifluormethylacetophenonoxim. Nach 15-minütigem Rühren des Reaktionsgemisches wird gesättigte Natriumhydrogencarbonatlösung zugegeben

und das Gemisch dreimal mit Aethylacetat extrahiert. Man trocknet die organische Phase über wasserfreiem Natriumsulfat und engt sie dann ein. Das zurückbleibende gelbe Oel wird an Kieselgel mit Diäthyläther/n-Hexan (1: 1) chromatographisch gereinigt und aus Diäthyläther/n-Hexan umkristallisiert. Auf diese Weise erhält man den α-[5-(α-Methyl-m-trifluormethyl-benzylidenaminooxymethyl)-3-benzo[b]thienyl]-β-methoxyacrylsäure-methylester als farblose Kristalle, Smp. 110-111 °C.

Beispiele 57-184

Analog dem in Beispiel 56 beschriebenen Verfahren wird α-(5-Brommethyl-3-benzo[b]thienyl)-β-methoxyacrylsäure-methylester mit dem jeweiligen Oxim der Formel III umgesetzt, um die in der nachstehenden Tabelle 3 aufgeführten Verbindungen der Formel I herzustellen:

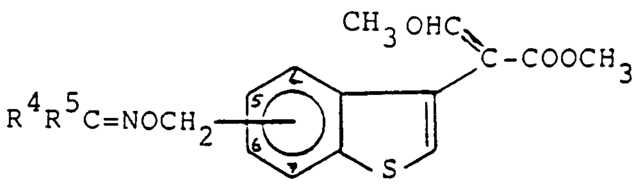

Tabelle 3

| Beispiel | Stellung von $R^4R^5C{=}NOCH_2$- | $R^4$ | $R^5$ | Physikalische Daten |
|---|---|---|---|---|
| 57 | 5- | Phenyl | Trifluormethyl | Oel |
| 58 | 5- | Phenyl | Methyl | Smp. 123°C |
| 59 | 5- | 4-Chlorphenyl | Methyl | Smp. 135-136°C |
| 60 | 5- | 2-Thienyl | Methyl | Smp. 111-112°C |
| 61 | 5- | m-Tolyl | Wasserstoff | Smp. 86-87°C |
| 62 | 5- | 3-Chlorphenyl | Methyl | Smp. 121-122°C |
| 63 | 5- | 3,4-Dichlorphenyl | Methyl | Smp. 149-150°C |
| 64 | 5- | 4-Nitrophenyl | Methyl | Smp. 161°C |
| 65 | 5- | Phenyl | n-Propyl | Oel |
| 66 | 5- | Cyclopropyl | Cyclopropyl | Oel |
| 67 | 5- | 2-Furyl | Methyl | Smp. 109°C |
| 68 | 5- | 2-Pyridyl | Methyl | Smp. 107-109°C |
| 69 | 5- | 3-Pyridyl | Methyl | Smp. 124-125°C |
| 70 | 5- | 4-Chlorphenyl | n-Propyl | Oel |
| 71 | 5- | 4-Chlorphenyl | Cyclopropyl | Oel |
| 72 | 5- | 3-Fluor-5-tri-fluormethyl-phenyl | Methyl | Smp. 92-93°C |
| 73 | 5- | 3,5-Difluorphenyl | Methyl | Smp. 129-130°C |
| 74 | 5- | 3-Fluorphenyl | Methyl | Smp. 129-130°C |
| 75 | 5- | 3-Trifluormethoxy-phenyl | Methyl | Smp. 75-76°C |
| 76 | 5- | 2,5-Dimethyl-3-furyl | Methyl | Smp. 78°C |
| 77 | 5- | 3-Thienyl | Methyl | Smp. 130-131°C |
| 78 | 5- | 2-Thienyl | Cyclopropyl | Oel |

| | | | | |
|---|---|---|---|---|
| 79 | 5- | 3-Methoxyphenyl | Methyl | Smp. 99°C |
| 80 | 5- | 4-tert.Butyl-phenyl | Methyl | Smp. 95°C |
| 81 | 5- | 4-Difluormethoxy-phenyl | Methyl | Smp. 98°C |
| 82 | 5- | α,α,α-Tri-fluor-m-tolyl | Trifluormethyl | Oel |
| 83 | 5- | α,α,α-Tri-fluor-m-tolyl | Aethyl | Oel |
| 84 | 5- | 3-Chlorphenyl | Aethyl | Smp. 81-82°C |
| 85 | 5- | α,α,α-Tri-fluor-m-tolyl | n-Propyl | Oel |
| 86 | 5- | α,α,α-Tri-fluor-m-tolyl | Methoxymethyl | Smp. 101-102°C |
| 87 | 5- | Methyl | Methyl | Smp. 102-103°C |
| 88 | 5- | Isopropyl | Methyl | Smp. 69-70°C |
| 89 | 5- | 1-Methyl-2-pyrrolyl | Methyl | Smp. 149°C |
| 90 | 5- | 2-Pyridyl | Cyclopropyl | Oel |
| 91 | 5- | 2-Thiazolyl | Cyclopropyl | 1. Isomeres: Smp. 146-147°C 2. Isomeres: Smp. 92-93°C |
| 92 | 5- | 3-Bromphenyl | Methyl | Smp. 112°C |
| 93 | 5- | α,α,α-Tri-fluor-m-tolyl | Cyclopropyl | Oel |
| 94 | 5- | 4-Fluorphenyl | Cyclopropyl | Smp. 97-99°C |
| 95 | 5- | 4-Pyridyl | Methyl | Smp. 159-160°C |
| 96 | 5- | Phenyl | Cyclopropyl | Oel |
| 97 | 5- | 3-Chlorphenyl | Cyclopropyl | Oel |
| 98 | 5- | 3-Trimethylsilyl-phenyl | Cyclopropyl | Oel |
| 99 | 5- | 2-Pyridyl | Isopropyl | Oel |
| 100 | 5- | 2-Pyridyl | Trifluormethyl | Smp. 110°C |
| 101 | 5- | 4-Fluor-3-tri-fluormethyl-phenyl | Cyclopropyl | Oel |
| 102 | 5- | 3-Fluor-5-trifluor-methyl-phenyl | Cyclopropyl | Oel |

| | | | | |
|---|---|---|---|---|
| 103 | 5- | 3-Fluorphenyl | Cyclopropyl | Oel |
| 104 | 5- | Cyclopropyl | Methyl | [E]-Isomeres: Smp. 112-114°C [Z]-Isomeres: Oel |
| 105 | 5- | 3-Bromphenyl | Cyclopropyl | Oel |
| 106 | 5- | n-Propyl | Methyl | Oel |
| 107 | 5- | Aethyl | Methyl | Oel |
| 108 | 5- | Isobutyl | Methyl | Oel |
| 109 | 5- | Cyclohexyl | Methyl | Oel |
| 110 | 5- | Benzyl | Methyl | Oel |
| 111 | 5- | α,α,α-Trifluor-m-tolyl | Wasserstoff | Smp. 117°C |
| 112 | 5- | α,α,α-Trifluor-m-tolyl | Isopropyl | Oel |
| 113 | 5- | 2-Methylthio-5-trifluormethyl-phenyl | Methyl | Oel |
| 114 | 5- | 4-Fluorphenyl | Methyl | Smp. 138-140°C |
| 115 | 5- | 3-Fluorphenyl | n-Propyl | Oel |
| 116 | 5- | 3,4-Dichlorphenyl | Wasserstoff | Smp. 157-159°C |
| 117 | 5- | 2-Pyridyl | Wasserstoff | Smp. 82°C |
| 118 | 5- | 3-Fluorphenyl | Wasserstoff | Smp. 112-114°C |
| 119 | 5- | p-Tolyl | Methyl | Smp. 116-117°C |
| 120 | 5- | 4-Methoxy-3-methylthiomethyl-phenyl | Methyl | Smp. 123-124°C |
| 121 | 5- | 3-Aethylphenyl | Methyl | Oel |
| 122 | 5- | α,α,α-Trifluor-p-tolyl | Wasserstoff | Smp. 165-166°C |
| 123 | 5- | 3-Bromphenyl | Methoxymethyl | Smp. 116-117°C |
| 124 | 5- | 4-Jodphenyl | Methyl | Smp. 144-145°C |
| 125 | 5- | Aethoxy | Methyl | Oel |
| 126 | 5- | Phenyl | Phenyl | Smp. 141-142°C |
| 127 | 5- | Benzoyl | Methyl | Smp. 95-96°C |
| 128 | 5- | 2-Thiazolyl | Wasserstoff | Smp. 215-217°C |
| 129 | 5- | 3-(3-Methyl-2-butenyloxy)-phenyl | Methyl | Oel |

| | | | | |
|---|---|---|---|---|
| 130 | 5- | 3-Trimethylsilyl-phenyl | Trifluormethyl | Oel |
| 131 | 5- | 2-Thienyl | tert. Butyl | Smp. 131°C |
| 132 | 5- | 3-(α,α,α-Trifluor-m-tolyl-oxy)-phenyl | Wasserstoff | Oel |
| 133 | 5- | 2-Thiazolyl | n-Propyl | Smp. 111-112°C |
| 134 | 5- | 2-Thienyl | n-Propyl | Oel |
| 135 | 5- | 5-(2,3-Dihydro-benzofuranyl) | Methyl | Oel |
| 136 | 5- | 6-(1,4-Benzo-dioxanyl) | Methyl | Oel |
| 137 | 5- | 2-Thienyl | Aethyl | Smp. 86-87°C |
| 138 | 5- | 2,5-Dimethyl-3-thienyl | Methyl | Smp. 110-111°C |
| 139 | 5- | 3-Benzyloxyphenyl | Methyl | Oel |
| 140 | 5- | 2-Thienyl | Wasserstoff | Oel |
| 141 | 5- | 3-Thienyl | Wasserstoff | Oel |
| 142 | 5- | Phenyl | Methylthiomethyl | Oel |
| 143 | 5- | Phenyl | Methoxymethyl | Oel |
| 144 | 5- | 2,5-Difluorphenyl | Methyl | Smp. 123-124°C |
| 145 | 5- | 3-Chlor-4-fluor-phenyl | Methyl | Smp. 148-150°C |
| 146 | 5- | p-(n-Propyl)-phenyl | Methyl | Smp. 99-100°C |
| 147 | 5- | 4-Fluor-3-trifluor-methyl-phenyl | Isopropyl | Oel |
| 148 | 5- | 4-Fluor-3-trifluor-methyl-phenyl | Methoxymethyl | Oel |
| 149 | 5- | 3-Bromphenyl | Isopropyl | Oel |
| 150 | 5- | 3-Chlorphenyl | Isopropyl | Oel |
| 151 | 5- | 3-Bromphenyl | n-Propyl | Oel |
| 152 | 5- | 3-Chlorphenyl | n-Propyl | Oel |
| 153 | 5- | 4-Fluor-3-trifluor-methyl-phenyl | n-Propyl | Oel |

| | | | | |
|---|---|---|---|---|
| | | 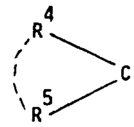 | | |
| 154 | 5- | 3-Methyl-5,6-dihydro-2H-1,4-thiazin-2-yliden | | Smp. 158-159°C |
| 155 | 5- | 3-Aethyl-5,6-dihydro-2H-1,4-thiazin-2-yliden | | Smp. 106°C |
| 156 | 5- | 3-Isopropyl-5,6-dihydro-2H-1,4-thiazin-2-yliden | | Smp. 119-120°C |
| 157 | 5- | 3-Cyclopropyl-5,6-dihydro-2H-1,4-thiazin-2-yliden | | Smp. 159-160°C |
| 158 | 5- | 3-Phenyl-5,6-dihydro-2H-1,4-thiazin-2-yliden | | Smp. 129-130°C |
| 159 | 5- | 2,4-Dimethyl-2,5-dihydro-thiazol-5-yliden | | Smp. 137-138°C |
| 160 | 5- | 2-Isopropyl-4-methyl-2,5-dihydro-thiazol-5-yliden | | Oel |
| 161 | 5- | 3-(4-Chlorphenyl)-5,6-dihydro-2H-1,4-thiazin-2-yliden | | Oel |
| 162 | 5- | 2-Aethyl-2,4-dimethyl-2,5-dihydro-thiazol-5-yliden | | Smp. 111-112°C |
| 163 | 5- | 2,2-Dimethyl-4-Isopropyl-2,5-dihydro-thiazol-5-yliden | | Smp. 113-114°C |
| 164 | 5- | 4,5,5-Trimethyl-2,5-dihydro-thiazol-2-yliden | | Smp. 145-147°C |
| 165 | 5- | Cyclopentyliden | | Oel |
| 166 | 5- | 1H-Indan-1-yliden | | Oel |
| 167 | 5- | Cyclohexyliden | | Oel |
| 168 | 5- | 9H-Fluoren-9-yliden | | Smp. 154°C |
| | | $R^4$ | $R^5$ | |
| 169 | 5- | p-Tolyl | Trifluormethyl | Oel |
| 170 | 5- | 3-Bromphenyl | Trifluormethyl | Oel |
| 171 | 5- | 3-Chlorphenyl | Trifluormethyl | Oel |
| 172 | 5- | 2,4-Dichlorphenyl | 2-Fluorphenyl | Oel |

| | | | | |
|---|---|---|---|---|
| 173 | 5- | 5-(1,3-Benzodioxolanyl) | Cyclopropyl | Oel |
| 174 | 5- | 5-Brom-2-thienyl | Methyl | Oel |
| 175 | 5- | 5-Methyl-2-furanyl | Methyl | Oel |
| 176 | 5- | 3-(α,α,α-Trifluor-m-tolyloxy)-phenyl | Methyl | Oel |
| 177 | 5- | 2-Chlorphenyl | Methyl | Oel |
| 178 | 5- | 4-Bromphenyl | Methyl | Smp. 132-133°C |
| 179 | 5- | 3-Phenoxyphenyl | Wasserstoff | Oel |
| 180 | 5- | p-Tolyl | Wasserstoff | Smp. 128-129°C |
| 181 | 5- | 4-Bromphenyl | Aethyl | Oel |
| 182 | 5- | 3,4-Dimethoxyphenyl | Methyl | Oel |
| 183 | 5- | 4-Aethylphenyl | Wasserstoff | Oel |
| 184 | 5- | 2,4-Dimethoxyphenyl | Methyl | Oel |

Beispiel 185

Eine Lösung von 10 g {3-(α-Methoxycarbonyl-β--methoxyvinyl) -5-benzo[b]-thienyl}methylphosphonsäure--dimethylester in 20 ml Tetrahydrofuran wird einer Suspension von 1,3 g Natriumhydrid in 30 ml Tetrahydrofuran zugetropft. Man gibt dann 3 ml Benzaldehyd hinzu und rührt das Reaktionsgemisch 2 Stunden bei Raumtemperatur. Anschliessend wird eingeengt, mit Wasser versetzt und mit Aethylacetat extrahiert. Nach Trocknen der organischen Phase über wasserfreiem Natriumsulfat und Einengen unter vermindertem Druck wird der Rückstand an Kieselgel mit Methylenchlorid/n-Hexan (1:1) chromatographisch gereinigt. Man erhält nach Umkristallisieren aus Methylenchlorid/Aethylacetat/n-Hexan den α-(5-Styryl-3-benzo[b]thienyl) -β-methoxyacrylsäure-methylester als farblose Kristalle, Smp. 180-181 °C.

Beispiele 186-188

Analog dem in Beispiel 185 beschriebenen Verfahren wird {3-(α-Methoxycarbonyl-β-methylvinyl)-5-benzo[b]-thienyl}methylphosphonsäure-dimethylester mit dem jeweiligen Aldehyd der Formel V umgesetzt, um die in der nachstehenden Tabelle 4 aufgeführten Verbindungen der Formel I herzustellen:

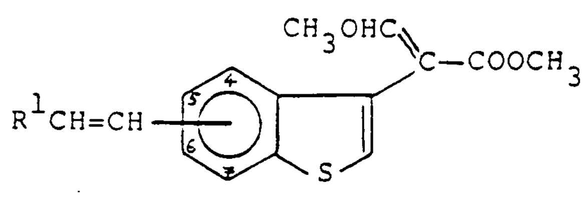

Tabelle 4

| Beispiel | Stellung $R^1CH=CH$- | von $R^1$ | Physikalische Daten |
|---|---|---|---|
| 186 | 5- | α,α,α-Trifluor-m-tolyl | Smp. 145-146 °C |
| 187 | 5- | 3-Thienyl | Smp. 214-215 °C |
| 188 | 5- | 2-Pyridyl | Smp. 126-127 °C |

Beispiel 189

Ein Gemisch von 0,9 g α-(5-Formyl-3-benzo[b]thienyl)--β-methoxyacrylsäure -methylester, 0,3 ml Anilin und einer katalytischen Menge p-Toluolsulfonsäure in 30 ml Aethanol wird 1 Stunde auf Rückflusstemperatur erhitzt. Dann engt man das Gemisch unter vermindertem Druck ein, gibt gesättigte Natriumhydrogencarbonatlösung hinzu und extrahiert dreimal mit Aethylacetat. Man trocknet die vereinigten, organischen Phasen über wasserfreiem Natriumsulfat und engt sie dann ein. Dabei erhält man den α-(5-Phenylimino-3-benzo[b]-thienyl)-β-methoxyacrylsäure -methylester als gelbliche Kristalle, Smp. 152 °C.

Beispiele 190-205

Analog dem in Beispiel 189 beschriebenen Verfahren wird α-(5-Formyl-3-benzo[b]thienyl)-β-methoxyacrylsäure-methylester mit dem jeweiligen Amin oder Hydroxylamin der Formel VII bzw. Hydrazin der Formel VIII umgesetzt, um die in den nachstehenden Tabellen 5 und 6 aufgeführten Verbindungen der Formel I herzustellen.

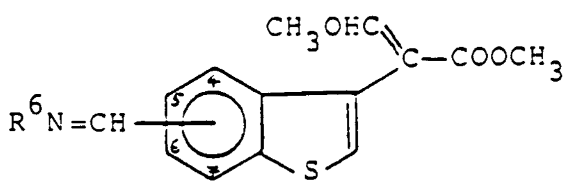

Tabelle 5

| Beispiel | Stellung von $R^6N=CH$- | $R^6$ | Physikalische Daten |
|---|---|---|---|
| 190 | 5- | Methoxy | Smp. 148-149 °C |
| 191 | 5- | Aethoxy | Smp. 98-99 °C |
| 192 | 5- | Allyloxy | Smp. 83-84 °C |
| 193 | 5- | Benzyloxy | Smp. 142-143 °C |
| 194 | 5- | 4-Chlorbenzyloxy | Smp. 134-135 °C |
| 195 | 5- | 3,4-Dichlorbenzyloxy | Smp. 142-143 °C |

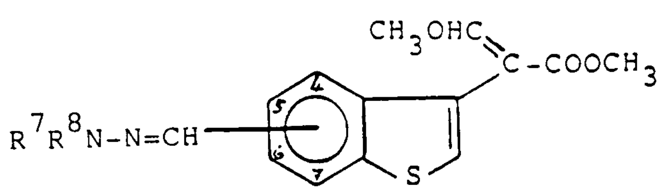

Tabelle 6

| Beispiel | Stellung von $R^7R^8N$-N=CH- | $R^7R^8N$ | Physikalische Daten |
|---|---|---|---|
| 196 | 5- | Dimethylamino | Smp. 172-173°C |
| 197 | 5- | Phenylamino | Smp. 229-233°C (unter Zersetzung) |
| 198 | 5- | p-Tolylsulfonylamino | Smp. 162-167°C (unter Zersetzung) |
| 199 | 5- | 6-Chlor-2-pyridylamino | Smp. 195-196°C |
| 200 | 5- | 3-Chlor-5-trifluormethyl-2-pyridylamino | Smp. 217-218°C |
| 201 | 5- | 2-Pyridylamino | Smp. 221-222°C |
| 202 | 5- | 7-Chlor-4-chinolinylamino | Smp. 159-161°C |
| 203 | 5- | 4,6-Dimethyl-2-pyrimidinyl-amino | Smp. 161°C |
| 204 | 5- | 6-Methyl-4-trifluormethyl-2-pyridylamino | Smp. 186-188°C |
| 205 | 5- | (Methyl)6-methyl-4-trifluor-methyl-2-pyridyl)amino | Smp. 225-226°C |

II. Herstellung der Ausgangsmaterialien der Formel IV:

Beispiel 206

Ein Gemisch von 20 g α-(5-Brommethyl-3-benzo[b]-thienyl) -β-methoxyacrylsäure-methylester (siehe Beispiel 11), 7,1 ml Trimethylester von phosphoriger Säure und 3,5 ml Toluol wird 2 Stunden auf Rückflusstemperatur erhitzt. Dann engt man das Gemisch unter vermindertem Druck ein und reinigt das verbleibende Oel chromatographisch an Kieselgel, und zwar zuerst mit Aethylacetat und anschliessend mit Aethylacetat/Methanol (9:1). Auf diese Weise erhält man den {3-(α-Methoxycarbonyl)-β-methoxyvinyl) -5-benzo[b]-thienyl}methylphosphonsäure-dimethylester als gelbes Oel.

III. Herstellung der Ausgangsmaterialien der Formel VI:

Beispiel 207

Ein Gemisch von 2 g α-(5-Brommethyl-3-benzo[b]-thienyl)-β-methoxyacrylsäure -methylester (siehe Beispiel 11), 16 ml Dimethylsulfoxid und 0,5 g Natriumhydrogencarbonat wird 2,5 Stunden bei 85 °C erhitzt. Anschliessend wird mit Wasser versetzt und mit Aethylacetat extrahiert. Nach Trocknen der organischen Phase über wasserfreiem Natriumsulfat und Einengen unter vermindertem Druck wird der Rückstand an Kieselgel mit Diäthyläther/n-Hexan (2:1) chromatographisch gereinigt. Man erhält nach Umkristallisieren aus

Methylenchlorid/n-Hexan den α-(5-Formyl-3-benzo[b]thienyl)-β-methoxyacrylsäure -methylester als farblose Kristalle, Smp. 134-135 °C.

IV. Formulierungsbeispiele:

Beispiel 208

Ein emulgierbares Konzentrat hat folgende Zusammensetzung:

| | g/Liter |
|---|---|
| Wirkstoff (erfindungsgemässe Verbindung) | 100 |
| Nonylphenol-(10)äthoxylat (nicht ionischer Emulgator) | 50 |
| Calcium-dodecylbenzolsulfonat (anionischer Emulgator) | 25 |
| N-Methyl-2-pyrrolidon (Lösungsvermittler) | 200 |
| Gemisch von Alkylbenzolen (Lösungsmittel) | ad 1 l |

Der Wirkstoff und die Emulgatoren werden im Lösungsmittel und im Lösungsvermittler gelöst. Durch Emulgieren dieses Konzentrates in Wasser kann eine gebrauchsfertige Spritzbrühe beliebiger Konzentration hergestellt werden.

Beispiel 209

Ein Spritzpulver hat folgende Zusammensetzung:

| | Gewichtsprozent |
|---|---|
| Wirkstoff (erfindungsgemässe Verbindung) | 25.0 |
| Kieselsäure (hydratisiert; Trägerstoff) | 20,0 |
| Natrium-laurylsulfat (Netzmittel) | 2,0 |
| Natrium-lignosulfonat (Dispergiermittel) | 4,0 |
| Kaolin (Trägerstoff) | 49,0 |

Die Komponenten werden miteinander vermischt, und das Ganze wird in einer geeigneten Mühle feingemahlen. Durch Dispergieren des Gemisches in Wasser ergibt sich eine Suspension, die sich als gebrauchsfertige Spritzbrühe eignet.

**Patentansprüche**

**Patentansprüche für folgende Vertragsstaaten : AT, BE, CH, DE, FR, GB, IT, LI, LU, NL**

**1.** Verbindungen der allgemeinen Formel

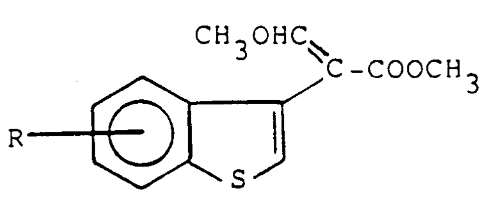

I

worin

R Halogen, $C_{1-4}$-Alkyl, $C_{1-4}$-Halogenalkyl, $C_{1-4}$-Alkoxy-$C_{1-4}$-alkyl, Aryl-$C_{1-4}$-alkoxy-$C_{1-4}$-alkyl, Aryloxy-$C_{1-4}$-alkyl, Heteroaryloxy-$C_{1-4}$-alkyl, Arylthio-$C_{1-4}$-alkyl, Heteroarylthio-$C_{1-4}$-alkyl,$C_{2-5}$-Alkanoyloxy-$C_{1-4}$-alkyl, (gegebenenfalls mit $C_{1-4}$-Alkyl substituiertes $C_{3-6}$-Cycloalkyl)carbonyloxy-$C_{1-4}$-alkyl, Aroyloxy-$C_{1-4}$-alkyl, Aryl-$C_{2-5}$-alkanoyloxy-$C_{1-4}$-alkyl, Heteroaryl-$C_{2-5}$-alkanoyloxy-$C_{1-4}$-alkyl, $C_{1-4}$-Alkoxy, Aryloxy oder eine der Gruppen (a) bis (e)

$R^1CH=CH$ (a)

$R^2R^3NCH_2$ (b)

$R^4R^5C=NOCH_2$ (c)

$R^6N=CH$ (d)

$R^7R^8N\text{-}N=CH$ (e)

$R^1$ Aryl oder Heteroaryl, $R^2$ und $R^3$ unabhängig voneinander Wasserstoff, $C_{1-4}$-Alkyl, Aryl-$C_{1-4}$-alkyl, Aryl oder Heteroaryl

oder

$R^2$ und $R^3$ zusammen mit dem Stickstoffatom, an das sie gebunden sind, einen 5- oder 6-gliedrigen, gegebenenfalls ein Sauerstoff- oder Schwefelatom enthaltenden Ring,

$R^4$ und $R^5$ unabhängig voneinander Wasserstoff, $C_{1-4}$-Alkyl, $C_{1-4}$-Halogenalkyl, $C_{1-4}$-Alkoxy-$C_{1-4}$-alkyl, $C_{1-4}$-Alkylthio-$C_{1-4}$-alkyl, Aryl-$C_{1-4}$-alkyl, $C_{3-6}$-Cycloalkyl, Aryl, Heteroaryl, $C_{1-4}$-Alkoxy oder Aroyl,

oder

$R^4$ und $R^5$ zusammen mit dem Kohlenstoffatom, an das sie gebunden sind, einen 5- oder 6-gliedrigen, gegebenenfalls einen oder zwei ankondensierte Benzolringe aufweisenden carbocyclischen oder heterocyclischen Ring,

$R^6$ Aryl, Heteroaryl, $C_{1-4}$-Alkoxy, Aryl-$C_{1-4}$-alkoxy oder $C_{3\ \text{oder}\ 4}$-Alkenyloxy,

und

$R^7$ und $R^8$ unabhängig voneinander Wasserstoff, $C_{1-4}$-Alkyl, Aryl, Heteroaryl oder Arylsulfonyl bedeuten.

**2.** Verbindungen nach Anspruch 1, worin R Halogen, $C_{1-4}$-Alkyl, $C_{1-4}$-halogenalkyl, $C_{1-4}$-Alkoxy-$C_{1-4}$-alkyl, Aryloxy-$C_{1-4}$-alkyl, Heteroaryloxy-$C_{1-4}$-alkyl, Arylthio-$C_{1-4}$-alkyl, Heteroarylthio-$C_{1-4}$-alkyl, $C_{1-4}$-Alkoxy, Aryloxy oder eine der Gruppen (a) bis (d) bedeutet, $R^1$, $R^2$ und $R^3$ die in Anspruch 1 angegebenen Bedeutungen besitzen, und $R^4$ und $R^5$ unabhängig voneinander Wasserstoff, $C_{1-4}$-Alkyl, $C_{1-4}$-Halogenalkyl, $C_{1-4}$-Alkoxy-$C_{1-4}$-alkyl, $C_{3-6}$-Cycloalkyl, Aryl oder Heteroaryl und $R^6$ Aryl oder Heteroaryl bedeuten.

**3.** Verbindungen nach Anspruch 1 oder 2, worin der Substituent R sich in der 5-Stellung des Benzo[b]-thiophenkerns befindet.

**4.** Verbindungen nach Anspruch 2, ausgewählt aus

α-{5-(α-Methyl-m-trifluormethyl-benzylidenaminooxymethyl)-3-benzo[b]thienyl}-β-methoxyacrylsäure-methylester,

α-{5-(α-[n-Propyl]-benzylidenaminooxymethyl)-3-benzo[b]thienyl}-β-methoxyacrylsäure-methylester,

α-{5-[α-(2-Pyridyl)-äthylidenaminooxymethyl]-3-benzo[b]thienyl}-β-methoxyacrylsäure-methylester,

α-{5-(α-Cyclopropyl-4-chlorbenzylidenaminooxymethyl)-3-benzo[b]thienyl}-β-methoxyacrylsäure-methylester,

α-{5-Methoxy-3-benzo[b]thienyl}-β-methoxyacrylsäure-methylester,

α-{5-Aethoxy-3-benzo[b]thienyl}-β-methoxyacrylsäure-methylester,

α-{5-[(α-Phenyläthyl)methylaminomethyl]-3-benzo[b]thienyl}-β-methoxyacrylsäure-methylester,

α-{5-(Dicyclopropylmethyldenaminooxymethyl)-3-benzo[b]thienyl}-β-methoxyacrylsäure-methylester,

α-{5-[(Cyclopropyl)(2-pyridyl)methylidenaminooxymethyl]-3-benzo[b]thienyl}-β-methoxyacrylsäure-methylester

und

α-{5-(α-Cyclopropyl-m-trifluormethyl-benzylidenaminooxymethyl)-3-benzo[b]thienyl}-β-methoxyacrylsäure-methylester.

**5.** Verbindungen nach Anspruch 1, ausgewählt aus:
$\alpha$-{5-($\alpha$-Cyclopropyl-benzylidenaminooxymethyl)-3-benzo[b]thienyl}-$\beta$-methoxyacrylsäure-methylester,
$\alpha$-{5-[$\beta$-Methyl-$\alpha$-(2-pyridyl)-propylidenaminooxymethyl]-3-benzo[b]thienyl}-$\beta$-methoxyacrylsäure-methylester
und
$\alpha$-{5-(2-Isopropyl-4-methyl-2,5-dihydro-thiazol-5-ylidenaminooxymethyl)-3-benzo[b]thienyl}-$\beta$-methoxyacrylsäure-methylester.

**6.** Fungizides Mittel, dadurch gekennzeichnet, dass es eine wirksame Menge mindestens einer Verbindung der allgemeinen Formel

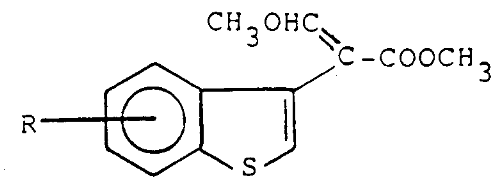

I

worin

| | |
|---|---|
| R | Halogen, $C_{1-4}$-Alkyl, $C_{1-4}$-Halogenalkyl, $C_{1-4}$-Alkoxy-$C_{1-4}$-alkyl, Aryl-$C_{1-4}$-alkoxy-$C_{1-4}$-alkyl, Aryloxy-$C_{1-4}$-alkyl, Heteroaryloxy-$C_{1-4}$-alkyl, Arylthio-$C_{1-4}$-alkyl, Heteroarylthio-$C_{1-4}$-alkyl,$C_{2-5}$-Alkanoyloxy-$C_{1-4}$-alkyl, (gegebenenfalls mit $C_{1-4}$-Alkyl substituiertes $C_{3-6}$-Cycloalkyl)carbonyloxy-$C_{1-4}$-alkyl, Aroyloxy-$C_{1-4}$-alkyl, Aryl-$C_{2-5}$-alkanoyloxy-$C_{1-4}$-alkyl, Heteroaryl-$C_{2-5}$-alkanoyloxy-$C_{1-4}$-alkyl, $C_{1-4}$-Alkoxy, Aryloxy oder eine der Gruppen (a) bis (e) |

$R^1CH =$ CH(a)

$R^2R^3NCH_2$ (b)

$R^4R^5C = NOCH_2$ (c)

$R^6N = CH$ (d)

$R^7R^8N\text{-}N = CH$ (e)

| | |
|---|---|
| $R^1$ | Aryl oder Heteroaryl, |
| $R^2$ und $R^3$ | unabhängig voneinander Wasserstoff, $C_{1-4}$-Alkyl, Aryl-$C_{1-4}$-alkyl, Aryl oder Heteroaryl |
| oder | |
| $R^2$ und $R^3$ | zusammen mit dem Stickstoffatom, an das sie gebunden sind, einen 5- oder 6-gliedrigen, gegebenenfalls ein Sauerstoff- oder Schwefelatom enthaltenden Ring, |
| $R^4$ und $R^5$ | unabhängig voneinander Wasserstoff, $C_{1-4}$-Alkyl, $C_{1-4}$-Halogenalkyl, $C_{1-4}$-Alkoxy-$C_{1-4}$-alkyl, $C_{1-4}$-Alkylthio-$C_{1-4}$-alkyl, Aryl-$C_{1-4}$-alkyl, $C_{3-6}$-Cycloalkyl, Aryl, Heteroaryl, $C_{1-4}$-Alkoxy oder Aroyl, |
| oder | |
| $R^4$ und $R^5$ | zusammen mit dem Kohlenstoffatom, an das sie gebunden sind, einen 5- oder 6-gliedrigen, gegebenenfalls einen oder zwei ankondensierte Benzolringe aufweisenden carbocyclischen oder heterocyclischen Ring, |
| $R^6$ | Aryl, Heteroaryl, $C_{1-4}$-Alkoxy, Aryl-$C_{1-4}$-alkoxy oder $C_{3\ \text{oder}\ 4}$-Alkenyloxy, |
| und | |
| $R^7$ und $R^8$ | unabhängig voneinander Wasserstoff, $C_{1-4}$-Alkyl, Aryl, Heteroaryl oder Arylsulfonyl bedeuten, |

sowie Formulierungshilfsstoffe enthält.

7. Fungizides Mittel nach Anspruch 6, dadurch gekennzeichnet, dass es eine wirksame Menge mindestens einer aus der Gruppe
α-{5-(α-Methyl-m-trifluormethyl-benzylidenaminooxymethyl)-3-benzo[b]thienyl}-3-methoxyacrylsäure-]methylester,
α-{5-(α-[n-Propyl]-benzylidenaminooxymethyl)-3-benzo[b]thienyl}-β-methoxyacrylsäure-methylester,
α-{5-[α-(2-Pyridyl)-äthylidenaminooxymethyl]-3-benzo[b]thienyl}-β-methoxyacrylsäure-methylester,
α-{5-(α-Cyclopropyl-4-chlorbenzylidenaminooxymethyl)-3-benzo[b]thienyl}-β-methoxyacrylsäure-methylester,
α-{5-Methoxy-3-benzo[b]thienyl}-β-methoxyacrylsäure-methylester,
α-{5-Aethoxy-3-benzo[b]thienyl}-β-methoxyacrylsäure-methylester,
α-{5-[α-Phenyläthyl)methylaminomethyl]-3-benzo[b]thienyl}-β-methoxyacrylsäure-methylester,
α-{5-(Dicyclopropylmethylidenaminooxymethyl)-3-benzo[b]thienyl}-β-methoxyacrylsäure-methylester,
α-{5-[(Cyclopropyl)(2-pyridyl)methylidenaminooxymethy]-3-benzo[b]thienyl}-β-methoxyacrylsäure-methylester und
α-{5-(α-Cyclopropyl-m-trifluormethyl-benzylidenaminooxymethyl)-3-benzo[b]thienyl}-β-methoxyacrylsäure-methylester ausgewählten Verbindung sowie Formulierungs hilfsstoffe enthält.

8. Fungizides Mittel nach Anspruch 6, dadurch gekennzeichnet, dass es eine wirksame Menge mindestens einer aus der Gruppe:
α-{-(α-Cyclopropyl-benzylidenaminooxymethyl)-3-benzo[b]thienyl}-β-methoxyacrylsäure-methylester,
α-{5-[β-Methyl-α-(2-pyridyl)-propylidenaminooxymethyl]-3-benzo[b]thienyl}-β-methoxyacrylsäure-methylester und
α-{5-(2-Isopropyl-4-methyl-2,5-dihydro-thiazol-5-ylidenaminooxymethyl)-3-benzo[b]thienyl}-β-methoxyacrylsäure-methylester
ausgewählten Verbindung sowie Formulierungshilfsstoffe enthält.

9. Verfahren zur Herstellung von Verbindungen der allgemeinen Formel

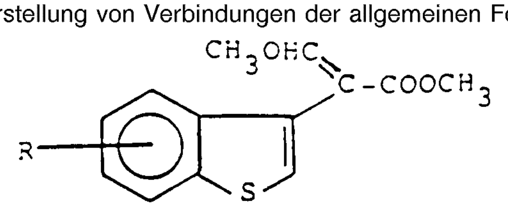

I

worin

R Halogen, $C_{1-4}$-Alkyl, $C_{1-4}$-Halogenalkyl, $C_{1-4}$-Alkoxy-$C_{1-4}$-alkyl, Aryl-$C_{1-4}$-alkoxy-$C_{1-4}$-alkyl, Aryloxy-$C_{1-4}$-alkyl, Heteroaryloxy-$C_{1-4}$-alkyl, Arylthio-$C_{1-4}$-alkyl, Heteroarylthio-$C_{1-4}$-alkyl,$C_{2-5}$-Alkanoyloxy-$C_{1-4}$-alkyl, (gegebenenfalls mit $C_{1-4}$-Alkyl substituiertes $C_{3-6}$-Cycloalkyl)carbonyloxy-$C_{1-4}$-alkyl, Aroyloxy-$C_{1-4}$-alkyl, Aryl-$C_{2-5}$-alkanoyloxy-$C_{1-4}$-alkyl, Heteroaryl-$C_{2-5}$-alkanoyloxy-$C_{1-4}$-alkyl, $C_{1-4}$-Alkoxy, Aryloxy oder eine der Gruppen (a) bis (e)

$R^1CH=CH$ (a)

$R^2R^3NCH_2$ (b)

$R^4R^5C=NOCH_2$ (c)

$R^6N=CH$ (d)

$R^7R^8N\text{-}N=CH$ (e)

$R^1$ Aryl oder Heteroaryl,

R² und R³ unabhängig voneinander Wasserstoff, $C_{1-4}$-Alkyl, Aryl-$C_{1-4}$-alkyl, Aryl oder Heteroaryl

oder

R² und R³ zusammen mit dem Stickstoffatom, an das sie gebunden sind, einen 5- oder 6-gliedrigen, gegebenenfalls ein Sauerstoff- oder Schwefelatom enthaltenden Ring,

R⁴ und R⁵ unabhängig voneinander Wasserstoff, $C_{1-4}$-Alkyl, $C_{1-4}$-Halogenalkyl, $C_{1-4}$-Alkoxy-$C_{1-4}$-alkyl, $C_{1-4}$-Alkylthio-$C_{1-4}$-alkyl, Aryl-$C_{1-4}$-alkyl, $C_{3-6}$-Cycloalkyl, Aryl, Heteroaryl, $C_{1-4}$-Alkoxy oder Aroyl,

oder

R⁴ und R⁵ zusammen mit dem Kohlenstoffatom, an das sie gebunden sind, einen 5- oder 6-gliedrigen, gegebenenfalls einen oder zwei ankondensierte Benzolringe aufweisenden carbocyclischen oder heterocyclischen Ring,

R⁶ Aryl, Heteroaryl, $C_{1-4}$-Alkoxy, Aryl-$C_{1-4}$-alkoxy oder $C_{3\ \text{oder}\ 4}$-Alkenyloxy,

und

R⁷ und R⁸ unabhängig voneinander Wasserstoff, $C_{1-4}$-Alkyl, Aryl, Heteroaryl oder Arylsulfonyl bedeuten,

dadurch gekennzeichnet, dass

a) zwecks Herstellung derjenigen Verbindungen der Formel I, in denen R verschieden von einer Gruppe (b), (c), (d) oder (e) ist, einen α-(3-Benzo[b]thienyl)-β-hydroxyacrylsäure-methylester der allgemeinen Formel

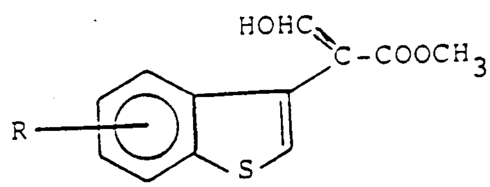

II

worin R die oben angegebene Bedeutung ausser einer Gruppe (b), (c), (d) oder (e) besitzt, mit einem Methylierungsmittel behandelt,

b) zwecks Herstellung derjenigen Verbindungen der Formel I, in denen R Brommethyl bedeutet, einen α-(Methyl-3-benzo[b]thienyl)-β-methoxyacrylsäure-methylester der allgemeinen Formel

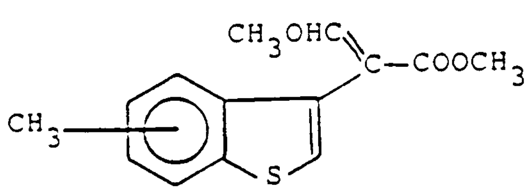

Ia

mit N-Bromsuccinimid behandelt,

c) zwecks Herstellung derjenigen Verbindungen der Formel I, in denen R $C_{1-4}$-Alkoxy-methyl, Aryl-$C_{1-4}$-alkoxy-methyl, Aryloxymethyl, Heteroaryloxymethyl, Arylthio-methyl, Heteroarylthiomethyl, $C_{2-5}$-Alkanoyloxy-methyl, (gegebenenfalls mit $C_{1-4}$-Alkyl substituiertes $C_{3-6}$-Cycloalkyl)-carbonyloxymethyl, Aroyloxymethyl, Aryl-$C_{2-5}$-alkanoyloxy-methyl, Heteroaryl-$C_{2-5}$-alkanoyloxy-methyl, eine Gruppe (b) oder eine Gruppe (c) bedeutet, einen α-(Brommethyl-3-benzo[b]thienyl)-β-methoxyacrylsäure-methylester der allgemeinen Formel

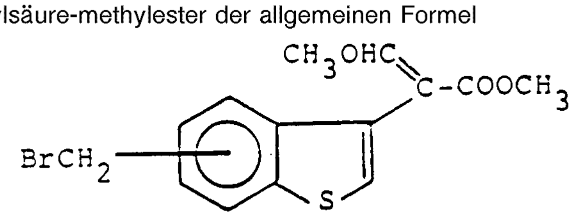

Ib

mit einer Hydroxyverbindung, einer Mercaptoverbindung, einer Carbonsäure, einem Amin bzw. einem Oxim der allgemeinen Formel

R'-H III

worin R′ $C_{1-4}$-Alkoxy, Aryl-$C_{1-4}$-alkoxy, Aryloxy, Heteroaryloxy, $C_{2-5}$-Alkanoyloxy, (gegebenenfalls mit $C_{1-4}$-Alkyl substituiertes $C_{3-6}$-Cycloalkyl)carbonyloxy, Aroyloxy, Aryl-$C_{2-5}$-alkanoyloxy, Heteroaryl-$C_{2-5}$-alkanoyloxy, Arylthio, Heteroarylthio, eine Gruppe $R^2R^3N$ (b′) oder eine Gruppe $R^4R^5C$ = NO (c′) bedeutet,
umsetzt,
d) zwecks Herstellung derjenigen Verbindungen der Formel I, in denen R eine Gruppe (a) bedeutet, einen {3-(α-Methoxycarbonyl-β-methoxyvinyl)-benzo [b]thienyl}methylphosphonsäure-dialkylester der allgemeinen Formel

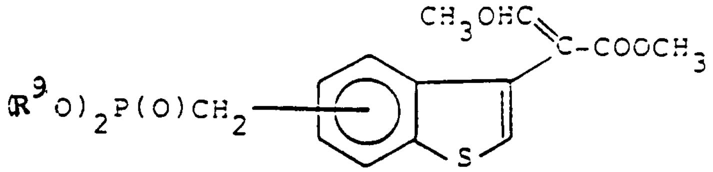
IV

worin $R^9$ $C_{1-4}$-Alkyl bedeutet, mit einem Aldehyd der allgemeinen Formel

$R^1CHO$ V

worin $R^1$ die oben angegebene Bedeutung besitzt, umsetzt, oder
e) zwecks Herstellung derjenigen Verbindungen der Formel I, in denen R eine Gruppe (d) oder eine Gruppe (e) bedeutet, einen α-(Formyl-3-benzo[b]thienyl)-β-methoxyacrylsäure-methylester der allgemeinen Formel

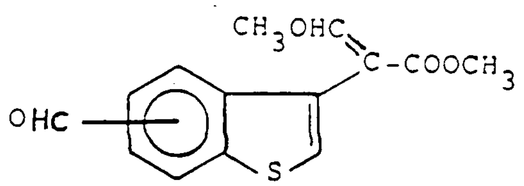
VI

mit einem Amin, Hydroxylamin bzw. Hydrazin der allgemeinen Formel

$R^6NH_2$ VII

bzw.

$R^7R^8N$-$NH_2$ VIII

worin $R^6$, $R^7$ und $R^8$ die oben angegebenen Bedeutungen besitzen,
umsetzt.

**10.** Verfahren nach Anspruch 9, dadurch gekennzeichnet, dass es zur Herstellung derjenigen Verbindungen der Formel I verwendet wird, in denen R Halogen, $C_{1-4}$-Alkyl, $C_{1-4}$-Halogenalkyl, $C_{1-4}$-Alkoxy-$C_{1-4}$-alkyl, Aryloxy-$C_{1-4}$-alkyl, Heteroaryloxy-$C_{1-4}$-alkyl, Arylthio-$C_{1-4}$-alkyl, Heteroarylthio-$C_{1-4}$-alkyl, $C_{1-4}$-Alkoxy, Aryloxy oder eine der in Anspruch 9 angegebenen Gruppen (a) bis (d) bedeutet, $R^1$, $R^2$ und $R^3$ die in Anspruch 9 angegebenen Bedeutungen besitzen, und $R^4$ und $R^5$ unabhängig voneinander Wasserstoff, $C_{1-4}$-Alkyl, $C_{1-4}$-Halogenalkyl, $C_{1-4}$-Alkoxy-$C_{1-4}$-alkyl, $C_{3-6}$-Cycloalkyl, Aryl oder Heteroaryl und $R^6$ Aryl oder Heteroaryl bedeuten.

**11.** Verfahren zur Bekämpfung von Fungi in der Landwirtschaft und im Gartenbau, dadurch gekennzeichnet, dass man das zu schützende Gut mit einer wirksamen Menge einer Verbindung gemäss einem der Ansprüche 1, 3 und 5 bzw. eines Mittels gemäss Anspruch 6 oder 8 behandelt.

12. Verfahren zur Bekämpfung von Fungi in der Landwirtschaft und im Gartenbau, dadurch gekennzeichnet, dass man das zu schützende Gut mit einer wirksamen Menge einer Verbindung gemäss Anspruch 2 oder 4 bzw. eines Mittels gemäss Anspruch 7 behandelt.

13. Verwendung einer Verbindung gemäss einem der Ansprüche 1, 3 und 5 bzw. eines Mittels gemäss Anspruch 6 oder 8 zur Bekämpfung von Fungi in der Landwirtschaft und im Gartenbau.

14. Verwendung einer Verbindung gemäss Anspruch 2 oder 4 bzw. eines Mittels gemäss Anspruch 7 zur Bekämpfung von Fungi in der Landwirtschaft und im Gartenbau.

**Patentansprüche für folgenden Vertragsstaat : ES**

1. Fungizides Mittel, dadurch gekennzeichnet, dass es eine wirksame Menge mindestens einer Verbindung der allgemeinen Formel

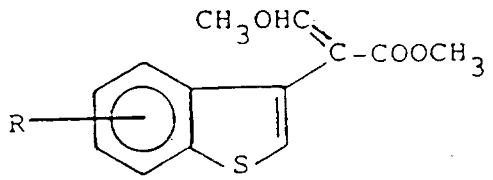

I

worin

R — Halogen, $C_{1-4}$-Alkyl, $C_{1-4}$-Halogenalkyl, $C_{1-4}$--Alkoxy-$C_{1-4}$-alkyl, Aryl-$C_{1-4}$-alkoxy-$C_{1-4}$-alkyl, Aryloxy-$C_{1-4}$-alkyl, Heteroaryloxy-$C_{1-4}$-alkyl, Arylthio-$C_{1-4}$-alkyl, Heteroarylthio-$C_{1-4}$-alkyl,$C_{2-5}$-Alkanoyloxy-$C_{1-4}$-alkyl. (gegebenenfalls mit $C_{1-4}$-Alkyl substituiertes $C_{3-6}$-Cycloalkyl)-carbonyloxy-$C_{1-4}$-alkyl, Aroyloxy-$C_{1-4}$-alkyl. Aryl-$C_{2-5}$ -alkanoyloxy-$C_{1-4}$-alkyl, Heteroaryl--$C_{2-5}$-alkanoyloxy-$C_{1-4}$-alkyl, $C_{1-4}$-Alkoxy, Aryloxy oder eine der Gruppen (a) bis (e)

$R^1CH=CH$ (a)

$R^2R^3NCH_2$ (b)

$R^4R^5C=NOCH_2$ (c)

$R^6N=CH$ (d)

$R^7R^8N\text{-}N=CH$ (e)

$R^1$ — Aryl oder Heteroaryl,

$R^2$ und $R^3$ — unabhängig voneinander Wasserstoff, $C_{1-4}$-Alkyl, Aryl-$C_{1-4}$-alkyl, Aryl oder Heteroaryl

oder

$R^2$ und $R^3$ — zusammen mit dem Stickstoffatom, an das sie gebunden sind, einen 5- oder 6-gliedrigen, gegebenenfalls ein Sauerstoff- oder Schwefelatom enthaltenden Ring.

$R^4$ und $R^5$ — unabhängig voneinander Wasserstoff, $C_{1-4}$-Alkyl, $C_{1-4}$-Halogenalkyl, $C_{1-4}$-Alkoxy-$C_{1-4}$-alkyl, $C_{1-4}$-Alkylthio-$C_{1-4}$-alkyl, Aryl-$C_{1-4}$-alkyl, $C_{3-6}$-Cycloalkyl, Aryl, Heteroaryl, $C_{1-4}$-Alkoxy oder Aroyl,

oder

$R^4$ und $R^5$ — zusammen mit dem Kohlenstoffatom, an das sie gebunden sind, einen 5- oder 6-gliedrigen, gegebenenfalls einen oder zwei ankondensierte Benzolringe aufweisenden carbocyclischen oder heterocyclischen Hing,

$R^6$ — Aryl, Heteroaryl, $C_{1-4}$-Alkoxy, Aryl-$C_{1-4}$-alkoxy oder $C_{3\ \text{oder}\ 4}$-Alkenyloxy,

und

$R^7$ und $R^8$ — unabhängig voneinander Wasserstoff, $C_{1-4}$-Alkyl, Aryl, Heteroaryl oder Arylsulfonyl bedeuten,

sowie Formulierungshilfsstoffe enthält.

2. Fungizides Mittel nach Anspruch 1 , dadurch gekennzeichnet, dass es eine wirksame Menge mindestens einer aus der Gruppe
α-{5-(α-Methyl-m-trifluormethyl-benzylidenaminooxymethyl)-3-benzo[b]thienyl}-β-methoxyacrylsäure-methylester,
α-{5-(α-[n-Propyl]-benzylidenaminooxymethyl)-3-benzo[b]thienyl}-β-methoxyacrylsäure-methylester,
α-{5-[α-(2-Pyridyl)-äthylidenaminooxymethyl]-3-benzo[b]thienyl}-β-methoxyacrylsäure-methylester,
α-{5-(α-Cyclopropyl-4-chlorbenzylidenaminooxymethyl)-3-benzo[b]thienyl}-β-methoxyacrylsäure-methylester,
α-{5-Methoxy-3-benzo[b]thienyl}-β-methoxyacrylsäure-methylester,
α-{5-Aethoxy-3-benzo[b]thienyl}-β-methoxyacrylsäure-methylester,
α-{5-[(α-Phenyläthyl)methylaminomethyl]-3-benzo-[b]thienyl}-β-methoxyacrylsäure-methylester,
α-{5-(Dicyclopropylmethylidenaminooxymethyl)-3-benzo[b]thienyl}-β-methoxyacrylsäure-methylester,
α-{5-[(Cyclopropyl)(2-pyridyl)methylidenaminooxymethyl]-3-benzo[b]thienyl]-β-methoxyacrylsäure-methylester und
α-{5(α-Cyclopropyl-m-trifluormethyl-benzylidenaminooxymethyl)-3-benzo[b]thienyl}-β-methoxyacrylsäure-methylester
ausgewählten Verbindung sowie Formulierungs- hilfsstoffe enthält.

3. Fungizides Mittel nach Anspruch 1 dadurch gekennzeichnet, dass es eine wirksame Menge mindestens einer aus der Gruppe:
α-{-(α-Cyclopropyl-benzylidenaminooxymethyl)-3-benzo[b]thienyl}-β-methoxyacrylsäure-methylester,
α-{5-[β-Methyl-α-(2-pyridyl)-propylidenaminooxymethyl]-3-benzo[b]thienyl}-β-methoxyacrylsäure-methylester und
α-{5-(2-Isopropyl-4-methyl-2,5-dihydro-thiazol-5-ylidenaminooxymethyl)-3-benzo[b]thienyl}-β-methoxyacrylsäure-methylester
ausgewählten Verbindung sowie Formulierungshilfsstoffe enthält.

4. Verfahren zur Herstellung von Verbindungen der allgemeinen Formel

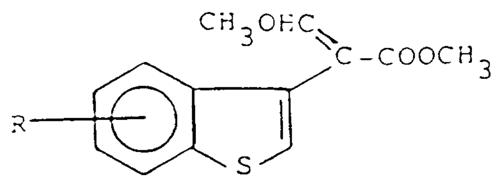

I

worin

R Halogen, $C_{1-4}$-Alkyl, $C_{1-4}$-Halogenalkyl, $C_{1-4}$--Alkoxy-$C_{1-4}$-alkyl, Aryl-$C_{1-4}$-alkoxy-$C_{1-4}$-alkyl, Aryloxy-$C_{1-4}$-alkyl, Heteroaryloxy-$C_{1-4}$-alkyl, Arylthio-$C_{1-4}$-alkyl, Heteroarylthio-$C_{1-4}$-alkyl,$C_{2-5}$-Alkanoyloxy-$C_{1-4}$-alkyl, (gegebenenfalls mit $C_{1-4}$-Alkyl substituiertes $C_{3-6}$-Cycloalkyl)-carbonyloxy-$C_{1-4}$-alkyl, Aroyloxy-$C_{1-4}$-alkyl, Aryl-$C_{2-5}$-alkanoyloxy-$C_{1-4}$-alkyl, Heteroaryl--$C_{2-5}$-alkanoyloxy-$C_{1-4}$-alkyl, $C_{1-4}$-Alkoxy, Aryloxy oder eine der Gruppen (a) bis (e)

$R^1CH = CH$ (a)

$R^2R^3NCH_2$ (b)

$R^4 R^5 C = NOCH_2$ (c)

$R^6 N = CH$ (d)

$R^7 R^8 N\text{-}N = CH$ (e)

$R^1$ Aryl oder Heteroaryl,

$R^2$ und $R^3$ unabhängig voneinander Wasserstoff, $C_{1-4}$-Alkyl, Aryl-$C_{1-4}$-alkyl, Aryl oder Heteroaryl

oder

$R^2$ und $R^3$ zusammen mit dem Stickstoffatom, an das sie gebunden sind, einen 5- oder 6-gliedrigen, gegebenenfalls ein Sauerstoff- oder Schwefelatom enthaltenden Ring,

$R^4$ und $R^5$ unabhängig voneinander Wasserstoff, $C_{1-4}$-Alkyl, $C_{1-4}$-Halogenalkyl, $C_{1-4}$-Alkoxy-$C_{1-4}$-alkyl, $C_{1-4}$-Alkylthio-$C_{1-4}$-alkyl, Aryl-$C_{1-4}$-alkyl, $C_{3-6}$-Cycloalkyl, Aryl, Heteroaryl, $C_{1-4}$-Alkoxy oder Aroyl,

oder

$R^4$ und $R^5$ zusammen mit dem Kohlenstoffatom, an das sie gebunden sind, einen 5- oder 6-gliedrigen, gegebenenfalls einen oder zwei ankondensierte Benzolringe aufweisenden carbocyclischen oder heterocyclischen Ring,

$R^6$ Aryl, Heteroaryl, $C_{1-4}$-Alkoxy, Aryl-$C_{1-4}$-alkoxy oder $C_{3\ \text{oder}\ 4}$-Alkenyloxy,

und

$R^7$ und $R^8$ unabhängig voneinander Wasserstoff, $C_{1-4}$-Alkyl, Aryl, Heteroaryl oder Arylsulfonyl bedeuten,

dadurch gekennzeichnet, dass

a) zwecks Herstellung derjenigen Verbindungen der Formel I, in denen R verschieden von einer Gruppe (b), (c), (d) oder (e) ist, einen α-(3-Benzo[b]thienyl)-β-hydroxyacrylsäure-methylester der allgemeinen Formel

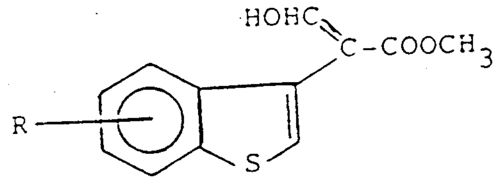

II

worin R die oben angegebene Bedeutung ausser einer Gruppe (b), (c), (d) oder (e) besitzt, mit einem Methylierungsmittel behandelt,

b) zwecks Herstellung derjenigen Verbindungen der Formel I, in denen R Brommethyl bedeutet, einen α-(Methyl-3--benzo[b]thienyl)-β-methoxyacrylsäure-methylester der allgemeinen Formel

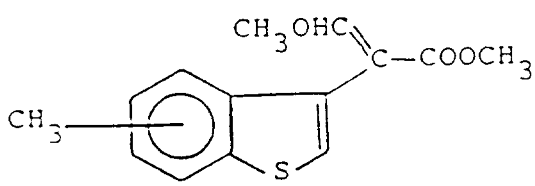

Ia

mit N-Bromsuccinimid behandelt,

c) zwecks Herstellung derjenigen Verbindungen der Formel I, in denen R $C_{1-4}$-Alkoxy-methyl, Aryl-$C_{1-4}$-alkoxy-methyl, Aryloxymethyl, Heteroaryloxymethyl, Arylthiomethyl, Heteroarylthiomethyl, $C_{2-5}$-Alkanoyloxy-methyl, (gegebenenfalls mit $C_{1-4}$-Alkyl substituiertes $C_{3-6}$-Cycloalkyl)-carbonyloxymethyl, Aroyloxymethyl, Aryl-$C_{2-5}$-alkanoyloxy-methyl, Heteroaryl-$C_{2-5}$-alkanoyloxy-methyl, eine Gruppe (b) oder eine Gruppe (c) bedeutet, einen α-(Brommethyl-3-benzo[b]thienyl)-β-methoxyacrylsäure-methylester der allgemeinen Formel

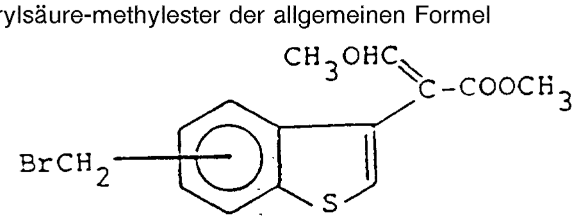

Ib

mit einer Hydroxyverbindung, einer Mercaptoverbindung, einer Carbonsäure, einem Amin bzw. einem Oxim der allgemeinen Formel

R'-H III

worin R' $C_{1-4}$-Alkoxy. Aryl-$C_{1-4}$-alkoxy, Aryloxy, Heteroaryloxy, $C_{2-5}$-Alkanoyloxy, (gegebenenfalls mit $C_{1-4}$-Alkyl substituiertes $C_{3-6}$-Cycloalkyl)-carbonyloxy, Aroyloxy, Aryl-$C_{2-5}$-alkanoyloxy, Heteroaryl-$C_{2-5}$-alkanoyloxy, Arylthio, Heteroarylthio, eine Gruppe $R^2R^3N$ (b') oder eine Gruppe $R^4R^5C=NO$ (c') bedeutet,
umsetzt,
d) zwecks Herstellung derjenigen Verbindungen der Formel I, in denen R eine Gruppe (a) bedeutet, einen {3-(α--Methoxycarbonyl -β-methoxyvinyl)-benzo[b]thienyl}methylphosphonsäure -dialkylester der allgemeinen Formel

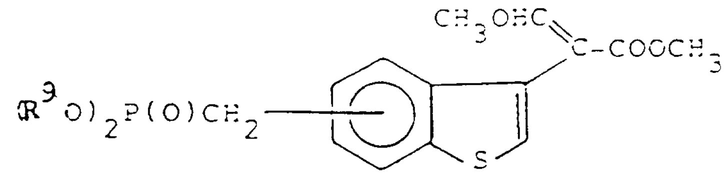

IV

worin $R^9$ $C_{1-4}$-Alkyl bedeutet, mit einem Aldehyd der allgemeinen Formel

$R^1CHO$ V

worin $R^1$ die oben angegebene Bedeutung besitzt, umsetzt, oder
e) zwecks Herstellung derjenigen Verbindungen der Formel I, in denen R eine Gruppe (d) oder eine Gruppe (e) bedeutet, einen α-(Formyl-3-benzo[b]thienyl)-β-methoxyacrylsäure--methylester der allgemeinen Formel

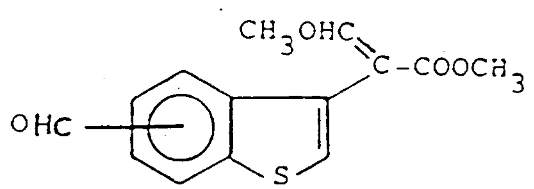

VI

mit einem Amin, Hydroxylamin bzw. Hydrazin der allgemeinen Formel

$R^6NH_2$ VII

bzw.

$R^7R^8N\text{-}NH_2$ VIII

worin $R^6$, $R^7$ und $R^8$ die oben angegebenen Bedeutungen besitzen,
umsetzt.

5. Verfahren nach Anspruch 4, dadurch gekennzeichnet, dass es zur Herstellung derjenigen Verbindungen der Formel I verwendet wird, in denen R Halogen, $C_{1-4}$-Alkyl, $C_{1-4}$--Halogenalkyl, $C_{1-4}$-Alkoxy-$C_{1-4}$-alkyl, Aryloxy--$C_{1-4}$-alkyl, Heteroaryloxy-$C_{1-4}$-alkyl, Arylthio-$C_{1-4}$--alkyl, Heteroarylthio-$C_{1-4}$-alkyl, $C_{1-4}$-Alkoxy, Aryloxy oder eine der in Anspruch 9 angegebenen Gruppen (a) bis (d) bedeutet, $R^1$, $R^2$ und $R^3$ die in Anspruch 9 angegebenen Bedeutungen besitzen, und $R^4$ und $R^5$ unabhängig voneinander Wasserstoff, $C_{1-4}$-Alkyl, $C_{1-4}$-Halogenalkyl, $C_{1-4}$-Alkoxy-$C_{1-4}$-alkyl, $C_{3-6}$-Cycloalkyl, Aryl oder Heteroaryl und $R^6$ Aryl oder Heteroaryl bedeuten.

6. Verfahren zur Bekämpfung von Fungi in der Landwirtschaft und im Gartenbau, dadurch gekennzeichnet, dass man das zu schützende Gut mit einer wirksamen Menge eines Mittels gemäss Anspruch 1

oder 3 behandelt.

7. Verfahren zur Bekämpfung von Fungi in der Landwirtschaft und im Gartenbau, dadurch gekennzeichnet, dass man das zu schützende Gut mit einer wirksamen Menge eines Mittels gemäss Anspruch 2 behandelt.

**Claims**

**Claims for the following Contracting States : AT, BE, CH, DE, FR, GB, IT, LI, LU, NL**

1. A compound of the general formula

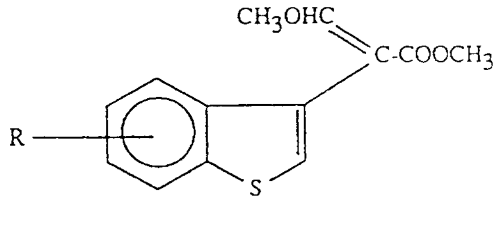

I

in which

R is halogen, $C_{1-4}$alkyl, $C_{1-4}$haloalkyl, $C_{1-4}$alkoxy-$C_{1-4}$alkyl, aryl-$C_{1-4}$alkoxy-$C_{1-4}$alkyl, aryloxy-$C_{1-4}$alkyl, heteroaryloxy-$C_{1-4}$alkyl, arylthio-$C_{1-4}$alkyl, heteroarylthio-$C_{1-4}$alkyl, $C_{2-5}$alkanoyloxy-$C_{1-4}$alkyl, (optionally $C_{1-4}$alkyl-substituted $c_{3-6}$cycloalkyl)-carbonyloxy-$C_{1-4}$alkyl, aroyloxy-$C_{1-4}$alkyl, aryl-$C_{2-5}$alkanoyloxy-$C_{1-4}$alkyl, heteroaryl-$C_{2-5}$alkanoyloxy-$C_{1-4}$alkyl, $C_{1-4}$alkoxy, aryloxy or one of the groups (a) to (e)

$R^1CH=CH$ (a)

$R^2R^3NCH_2$ (b)

$R^4R^5C=NOCH_2$ (c)

$R^6N=CH$ (d)

$R^7R^8N\text{-}N=CH$ (e),

$R^1$ is aryl or heteroaryl,
$R^2$ and $R^3$ independently of one another are hydrogen, $C_{1-4}$alkyl, aryl-$C_{1-4}$alkyl, aryl or heteroaryl,
or
$R^2$ and $R^3$ together with the nitrogen atom to which they are bonded are a 5- or 6-membered ring which may contain an oxygen or sulfur atom, $R^4$ and $R^5$ independently of one another are hydrogen, $C_{1-4}$alkyl, $C_{1-4}$haloalkyl, $C_{1-4}$alkoxy-$C_{1-4}$alkyl, $C_{1-4}$alkylthio-$C_{1-4}$alkyl, aryl-$C_{1-4}$alkyl, $C_{3-6}$cycloalkyl, aryl, heteroaryl, $C_{1-4}$alkoxy or aroyl,
or
$R^4$ and $R^5$ together with the carbon atom to which they are bonded are a 5-or 6-membered carbocyclic or heterocyclic ring to which one or two benzene rings may be fused,
$R^6$ is aryl, heteroaryl, $C_{1-4}$alkoxy, aryl-$C_{1-4}$alkoxy or $C_{3\ or\ 4}$alkenyloxy,
and
$R^7$ and $R^8$ independently of one another are hydrogen, $C_{1-4}$alkyl, aryl, heteroaryl or arylsulfonyl.

2. A compound according to claim 1, in which R is halogen, $C_{1-4}$alkyl, $C_{1-4}$haloalkyl, $C_{1-4}$alkoxy-$C_{1-4}$alkyl, aryloxy-$C_{1-4}$alkyl, heteroaryloxy-$C_{1-4}$alkyl, arylthio-$C_{1-4}$alkyl, heteroarylthio-$C_{1-4}$alkyl, $C_{1-4}$alkoxy, aryloxy or one of the groups (a) to (d), $R^1$, $R^2$ and $R^3$ are as defined in claim 1, and $R^4$ and $R^5$ independently of one another are hydrogen, $C_{1-4}$alkyl, $C_{1-4}$haloalkyl, $C_{1-4}$alkoxy-$C_{1-4}$alkyl, $C_{3-6}$cycloalkyl, aryl or heteroaryl, and $R^6$ is aryl or heteroaryl.

3. A compound according to claim 1 or 2, in which the substituent R is in the 5-position of the benzo[b]-thiophene ring.

4. A compound according to claim 2, selected from amongst methyl α-{5-(α-methyl-m-trifluoromethylbenzylideneaminooxymethyl)-3-benzo[b]thienyl}-β-methoxyacrylate,
methyl α-{5-(α-[n-propyl]-benzylideneaminooxymethyl)-3-benzo[b]thienyl}-β-methoxyacrylate,
methyl α-{5[α-(2-pyridyl)-ethylideneaminooxymethyl]-3-benzo[b]thienyl}-β-methoxyacrylate,
methyl α-{5-(α-cyclopropyl-4-chlorobenzylideneaminooxymethyl)-3-benzo[b]thienyl}-β-methoxyacrylate,
methyl α-{5-methoxy-3-benzo[b]thienyl}-β-methoxyacrylate,
methyl α-{5-ethoxy-3-benzo[b]thienyl}-β-methoxyacrylate,
methyl α-{5-[(α-phenylethyl)methylaminomethyl]-3-benzo[b]thienyl}-β-methoxyacrylate,
methyl α-{5-(dicyclopropylmethylideneaminooxymethyl)-3-benzo[b]thienyl}-β-methoxyacrylate,
methyl α-{5-[(cyclopropyl)(2-pyridyl)methylideneaminooxymethyl]-3-benzo[b]thienyl}-β-methoxyacrylate and
methyl α-{5-(α-cyclopropyl-m-trifluoromethylbenzylideneaminooxymethyl)-3-benzo[b]thienyl}-β-methoxyacrylate.

5. A compound according to claim 1, selected from amongst:
methyl α-{5-(α-cyclopropylbenzylideneaminooxymethyl)-3-benzo[b]thienyl}-β-methoxyacrylate,
methyl α-{5-[β-methyl-α-(2-pyridyl)-propylideneaminooxymethyl]-3-benzo[b]thienyl}-β-methoxyacrylate and
methyl α-{5-(2-isopropyl-4-methyl-2,5-dihydrothiazol-5-ylideneaminooxymethyl)-3-benzo[b]thienyl}-β-methoxyacrylate.

6. A fungicidal composition which contains an effective amount of at least one compound of the general formula

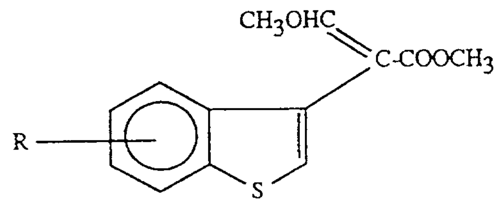

I

in which
R is halogen, $C_{1-4}$alkyl, $C_{1-4}$haloalkyl, $C_{1-4}$alkoxy-$C_{1-4}$alkyl, aryl-$C_{1-4}$alkoxy-$C_{1-4}$alkyl, aryloxy-$C_{1-4}$alkyl, heteroaryloxy-$C_{1-4}$alkyl, arylthio-$C_{1-4}$alkyl, heteroarylthio-$C_{1-4}$alkyl, $C_{2-5}$alkanoyloxy-$C_{1-4}$alkyl, (optionally $C_{1-4}$alkyl-substituted $C_{3-6}$cycloalkyl)-carbonyloxy-$C_{1-4}$alkyl, aroyloxy-$C_{1-4}$alkyl, aryl-$C_{2-5}$alkanoyloxy-$C_{1-4}$alkyl, heteroaryl-$C_{2-5}$alkanoyloxy-$C_{1-4}$alkyl, $C_{1-4}$alkoxy, aryloxy or one of the groups (a) to (e)

$R^1CH=CH$ (a)

$R^2R^3NCH_2$ (b)

$R^4R^5C=NOCH_2$ (c)

$R^6N=CH$ (d)

$R^7R^8N\text{-}N=CH$ (e),

$R^1$ is aryl or heteroaryl,
$R^2$ and $R^3$ independently of one another are hydrogen, $C_{1-4}$alkyl, aryl-$C_{1-4}$alkyl, aryl or heteroaryl,
or
$R^2$ and $R^3$ together with the nitrogen atom to which they are bonded are a 5- or 6-membered ring which may contain an oxygen or sulfur atom,
$R^4$ and $R^5$ independently of one another are hydrogen, $C_{1-4}$alkyl, $C_{1-4}$haloalkyl, $C_{1-4}$alkoxy-$C_{1-4}$alkyl, $C_{1-4}$alkylthio-$C_{1-4}$alkyl, aryl-$C_{1-4}$alkyl, $C_{3-6}$cycloalkyl, aryl, heteroaryl, $C_{1-4}$alkoxy or aroyl,
or
$R^4$ and $R^5$ together with the carbon atom to which they are bonded are a 5-or 6-membered carbocyclic

or heterocyclic ring to which one or two benzene rings may be fused,
$R^6$ is aryl, heteroaryl, $C_{1-4}$alkoxy, aryl-$C_{1-4}$alkoxy or $C_{3\ or\ 4}$lkenyloxy,
and
$R^7$ and $R^8$ independently of one another are hydrogen, $C_{1-4}$alkyl, aryl, heteroaryl or arylsulfonyl,
and also formulation auxiliaries.

**7.** A fungicidal composition according to claim 6, which contains an effective amount of at least one compound selected from the group methyl α-{5-(α-methyl-m-trifluoromethylbenzylideneaminooxymethyl)-3-benzo[b]thienyl}-β-methoxyacrylate,
methyl α-{5-(α-[n-propyl]-benzylideneaminooxymethyl)-3-benzo[b]thienyl}-β-methoxyacrylate,
methyl α-{5-[α-(2-pyridyl)-ethylideneaminooxymethyl]-3-benzo[b]thienyl}-β-methoxyacrylate,
methyl α-{5-(α-cyclopropyl-4-chlorobenzylideneaminooxymethyl)-3-benzo[b]thienyl}-β-methoxyacrylate,
methyl α-{5-methoxy-3-benzo[b]thienyl}-β-methoxyacrylate,
methyl α-{5-ethoxy-3-benzo[b]thienyl}-β-methoxyacrylate,
methyl α-{5-[(α-phenylethyl)methylaminomethyl]-3-benzo[b]thienyl}-β-methoxyacrylate,
methyl α-{5-(dicyclopropylmethylideneaminooxymethyl)-3-benzo[b]thienyl}-β-methoxyacrylate,
methyl α-{5-[(cyclopropyl)(2-pyridyl)methylideneaminooxymethyl]-3-benzo[b]thienyl}-β-methoxyacrylate and
methyl α-{5-(α-cyclopropyl-m-trifluoromethylbenzylideneaminooxymethyl)-3-benzo[b]thienyl}-β-methoxyacrylate,
and also formulation auxiliaries.

**8.** A fungicidal composition according to claim 6, which contains an effective amount of at least one compound selected from the group: methyl α-[5[(α-cyclopropylbenzylideneaminooxymethyl)-3-benzo-[b]thienyl}-β-methoxyacrylate, methyl α-{5[β-methyl-α-(2-pyridyl)-propylideneaminooxymethyl]-3-benzo[b]thienyl}-β-methoxyacrylate and
methyl α-{5-(2-isopropyl-4-methyl-2,5-dihydrothiazol-5-ylideneaminooxymethyl)-3-benzo[b]thienyl}-β-methoxyacrylate, and also formulation auxiliaries.

**9.** A process for the preparation of a compound of the general formula

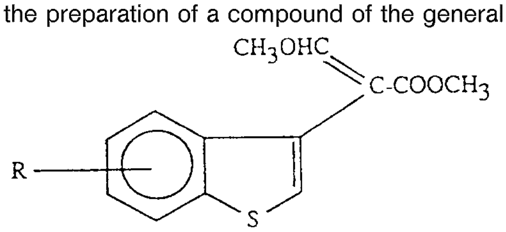

I

in which
R is halogen, $C_{1-4}$alkyl, $C_{1-4}$haloalkyl, $C_{1-4}$alkoxy-$C_{1-4}$alkyl, aryl-$C_{1-4}$alkoxy-$C_{1-4}$alkyl, aryloxy-$C_{1-4}$alkyl, heteroaryloxy-$C_{1-4}$alkyl, arylthio-$C_{1-4}$alkyl, heteroarylthio-$C_{1-4}$alkyl, $C_{2-5}$alkanoyloxy-$C_{1-4}$alkyl, (optionally $C_{1-4}$alkyl-substituted $C_{3-6}$cycloalkyl)-carbonyloxy-$C_{1-4}$alkyl, aroyloxy-$C_{1-4}$alkyl, aryl-$C_{2-5}$alkanoyloxy-$C_{1-4}$alkyl, heteroaryl-$C_{2-5}$alkanoyloxy-$C_{1-4}$alkyl, $C_{1-4}$alkoxy, aryloxy or one of the groups (a) to (e)

$R^1CH=CH$ (a)

$R^2R^3NCH_2$ (b)

$R^4R^5C=NOCH_2$ (c)

$R^6N=CH$ (d)

$R^7R^8N\text{-}N=CH$ (e),

$R^1$ is aryl or heteroaryl,
$R^2$ and $R^3$ independently of one another are hydrogen, $C_{1-4}$alkyl, aryl-$C_{1-4}$alkyl, aryl or heteroaryl,

or
$R^2$ and $R^3$ together with the nitrogen atom to which they are bonded are a 5- or 6-membered ring which may contain an oxygen or sulfur atom,
$R^4$ and $R^5$ independently of one another are hydrogen, $C_{1-4}$alkyl, $C_{1-4}$haloalkyl, $C_{1-4}$alkoxy-$C_{1-4}$alkyl, $C_{1-4}$alkylthio-$C_{1-4}$alkyl, aryl-$C_{1-4}$alkyl,
$C_{3-6}$cycloalkyl, aryl, heteroaryl, $C_{1-4}$alkoxy or aroyl,
or
$R^4$ and $R^5$ together with the carbon atom to which they are bonded are a 5-or 6-membered carbocyclic or heterocyclic ring to which one or two benzene rings may be fused,
$R^6$ is aryl, heteroaryl, $C_{1-4}$alkoxy, aryl-$C_{1-4}$alkoxy or $C_{3\text{ or }4}$alkenyloxy,
and
$R^7$ and $R^8$ independently of one another are hydrogen, $C_{1-4}$alkyl, aryl, heteroaryl or arylsulfonyl, which comprises,

a) to prepare those compounds of the formula I in which R is other than a group (b), (c), (d) or (e), treating a methyl α-(3-benzo[b]thienyl)-β-hydroxyacrylate of the general formula

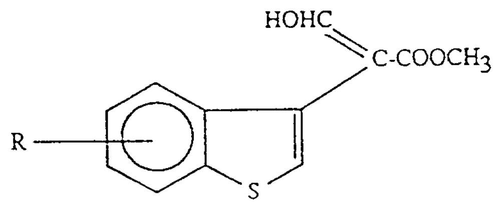

II

in which R is as defined above, with the exception of a group (b), (c), (d) or (e), with a methylating agent,

b) to prepare those compounds of the formula I in which R is bromomethyl, treating a methyl α-(methyl-3-benzo[b]thienyl)-β-methoxyacrylate of the general formula

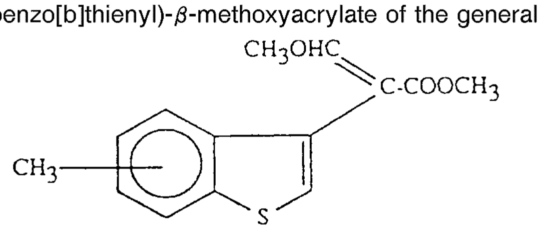

Ia

with N-bromosuccinimide,

c) to prepare those compounds of the formula I in which R is $C_{1-4}$alkoxy-methyl, aryl-$C_{1-4}$alkoxymethyl, aryloxymethyl, heteroaryloxymethyl, arylthiomethyl, heteroarylthiomethyl, $C_{2-5}$alkanoyloxymethyl, (optionally $C_{1-4}$alkyl-substituted $C_{3-6}$cycloalkyl)carbonyloxymethyl, aroyloxymethyl, aryl-$C_{2-5}$alkanoyloxymethyl, heteroaryl-$C_{2-5}$alkanoyloxymethyl, a group (b) or a group (c), reacting a methyl α-(bromomethyl-3-benzo[b]thienyl)-β-methoxyacrylate of the general formula

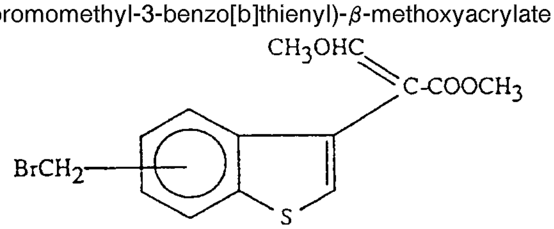

Ib

with a hydroxy compound, a mercapto compound, a carboxylic acid, an amine or an oxime of the general formula

R'-H III

in which R' is $C_{1-4}$alkoxy, aryl-$C_{1-4}$alkoxy, aryloxy, heteroaryloxy, $C_{2-5}$alkanoyloxy, (optionally $C_{1-4}$alkyl-substituted $C_{3-6}$cycloalkyl)-carbonyloxy, aroyloxy, aryl-$C_{2-5}$alkanoyloxy, heteroaryl-$C_{2-5}$alkanoyloxy, arylthio, heteroarylthio, a group $R^2R^3N$ (b') or a group $R^4R^5C=NO$ (c'),

d) to prepare those compounds of the formula I in which R is a group (a), reacting a dialkyl {3-(α-methoxycarbonyl-β-methoxyvinyl)-benzo[b]-thienyl}methylphosphonate of the general formula

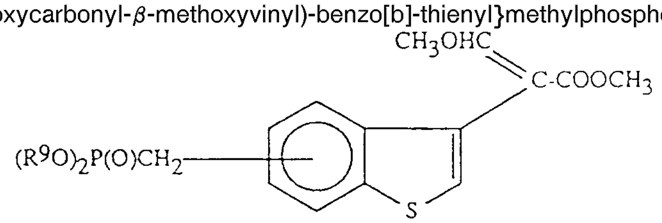

IV

in which $R^9$ is $C_{1-4}$alkyl, with an aldehyde of the general formula

$R^1CHO$ V

in which $R^1$ has the abovementioned meaning, or

e) to prepare those compounds of the formula I in which R is a group (d) or a group (e), reacting a methyl α-(formyl-3-benzo[b]thienyl)-β-methoxyacrylate of the general formula

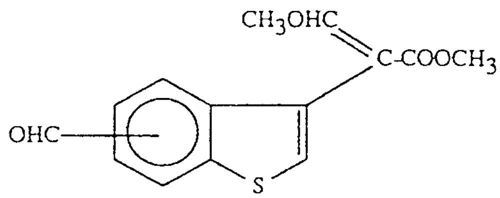

VI

with an amine, hydroxylamine or hydrazine of the general formula

$R^6NH_2$ VII

or

$R^7R^8N\text{-}NH_2$ VIII

in which $R^6$, $R^7$ and $R^8$ are as defined above.

**10.** The process according to claim 9, which is used for preparing those compounds of the formula I in which R is halogen, $C_{1-4}$alkyl, $C_{1-4}$haloalkyl, $C_{1-4}$alkoxy-$C_{1-4}$alkyl, aryloxy-$C_{1-4}$alkyl, heteroaryloxy-$C_{1-4}$alkyl, arylthio-$C_{1-4}$alkyl, heteroarylthio-$C_{1-4}$alkyl, $C_{1-4}$alkoxy, aryloxy or one of the groups (a) to (d) defined in claim 9, $R^1$, $R^2$ and $R^3$ are as defined in claim 9, and $R^4$ and $R^5$ independently of one another are hydrogen, $C_{1-4}$alkyl, $C_{1-4}$haloalkyl, $C_{1-4}$alkoxy-$C_{1-4}$alkyl, $C_{3-6}$cycloalkyl, aryl or heteroaryl, and $R^6$ is aryl or heteroaryl.

**11.** A method of controlling fungi in agriculture and in horticulture, which comprises treating the goods to be protected with an effective amount of a compound according to any one of claims 1, 3 and 5, or of a composition according to claim 6 or 8.

**12.** A method of controlling fungi in agriculture and in horticulture, which comprises treating the goods to be protected with an effective amount of a compound according to claim 2 or 4, or of a composition according to claim 7.

**13.** The use of a compound according to any one of claims 1, 3 and 5, or of a composition according to claim 6 or 8, for controlling fungi in agriculture and in horticulture.

**14.** The use of a compound according to claim 2 or 4, or of a composition according to claim 7, for controlling fungi in agriculture and in horticulture.

**Claims for the following Contracting State : ES**

1. A fungicidal composition which contains an effective amount of at least one compound of the general formula

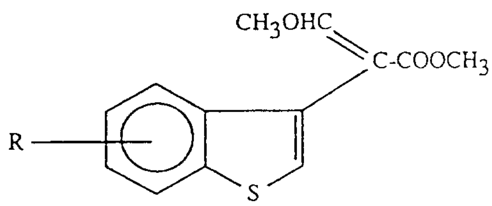

I

in which
R is halogen, $C_{1-4}$alkyl, $C_{1-4}$haloalkyl, $C_{1-4}$alkoxy-$C_{1-4}$alkyl, aryl-$C_{-14}$alkoxy-$C_{1-4}$alkyl, aryloxy-$C_{1-4}$alkyl, heteroaryloxy-$C_{1-4}$alkyl, arylthio-$C_{1-4}$alkyl, heteroarylthio-$C_{1-4}$alkyl, $C_{2-5}$alkanoyloxy-$C_{1-4}$alkyl, (optionally $C_{1-4}$alkyl-substituted $C_{3-6}$cycloalkyl)-carbonyloxy-$C_{1-4}$alkyl, aroyloxy-$C_{1-4}$alkyl, aryl-$C_{2-5}$alkanoyloxy-$C_{1-4}$alkyl, heteroaryl-$C_{2-5}$alkanoyloxy-$C_{1-4}$alkyl, $C_{1-4}$alkoxy, aryloxy or one of the groups (a) to (e)

$R^1CH=CH$ (a)

$R^2R^3NCH_2$ (b)

$R^4R^5C=NOCH_2$ (c)

$R^6N=CH$ (d)

$R^7R^8N\text{-}N=CH$ (e),

$R^1$ is aryl or heteroaryl,
$R^2$ and $R^3$ independently of one another are hydrogen, $C_{1-4}$alkyl, aryl-$C_{1-4}$alkyl, aryl or heteroaryl,
or
$R^2$ and $R^3$ together with the nitrogen atom to which they are bonded are a 5- or 6-membered ring which may contain an oxygen or sulfur atom,
$R^4$ and $R^5$ independently of one another are hydrogen, $C_{1-4}$alkyl, $C_{1-4}$haloalkyl, $C_{1-4}$alkoxy-$C_{1-4}$alkyl, $C_{1-4}$alkylthio-$C_{1-4}$alkyl, aryl-$C_{1-4}$alkyl, $C_{3-6}$cycloalkyl, aryl, heteroaryl, $C_{1-4}$alkoxy or aroyl,
or
$R^4$ and $R^5$ together with the carbon atom to which they are bonded are a 5-or 6-membered carbocyclic or heterocyclic ring to which one or two benzene rings may be fused,
$R^6$ is aryl, heteroaryl, $C_{1-4}$alkoxy, aryl-$C_{1-4}$alkoxy or $C_{3\text{ or }4}$alkenyloxy,
and
$R^7$ and $R^8$ independently of one another are hydrogen, $C_{1-4}$alkyl, aryl, heteroaryl or arylsulfonyl,
and also formulation auxiliaries.

2. A fungicidal composition according to claim 1, which contains an effective amount of at least one compound selected from the group methyl α-{5-(α-methyl-m-trifluoromethylbenzylideneaminooxymethyl)-3-benzo[b]thienyl)-β-methoxyacrylate,
methyl α-{5-(α-[n-propyl]-benzylideneaminooxymethyl)-3-benzo[b]thienyl}-β-methoxyacrylate,
methyl α-{5[α-(2-pyridyl)-ethylideneaminooxymethyl]-3-benzo[b]thienyl}-β -methoxyacrylate,
methyl α-{5-(α-cyclopropyl-4-chlorobenzylideneaminooxymethyl)-3-benzo[b]thienyl}-β-methoxyacrylate,
methyl α-{5-methoxy-3-benzo[b]thienyl}-β-methoxyacrylate,
methyl α-{5-ethoxy-3-benzo[b]thienyl}-β-methoxyacrylate,
methyl α-{5-[(α-phenylethyl)methylaminomethyl]-3-benzo[b]thienyl}-β-methoxyacrylate,
methyl α-{5-(dicyclopropylmethylideneaminooxymethyl)-3-benzo[b]thienyl}-β-methoxyacrylate,
methyl α-{5-[(cyclopropyl)(2-pyridyl)methylideneaminooxymethyl]-3-benzo[b]thienyl}-β-methoxyacrylate and
methyl α-{5-(α-cyclopropyl-m-trifluoromethylbensylideneaminooxymethyl)-3-benzo[b]thienyl}-β-methoxyacrylate,

and also formulation auxiliaries.

3. A fungicidal composition according to claim 1, which contains an effective amount of at least one compound selected from the group: methyl α-{5-(α-cyclopropylbenzylideneaminooxymethyl)-3-benzo-[b]thienyl}-β-methoxyacrylate,
methyl α-{5-[β-methyl-α-(2-pyridyl)-propylideneaminooxymethyl]-3-benzo[b]thienyl}-β-methoxyacrylate and
methyl α-{5-(2-isopropyl-4-methyl-2,5-dihydrothiazol-5-ylideneaminooxymethyl)-3-benzo[b]thienyl}-β-methoxyacrylate, and also formulation auxiliaries.

4. A process for the preparation of a compound of the general formula

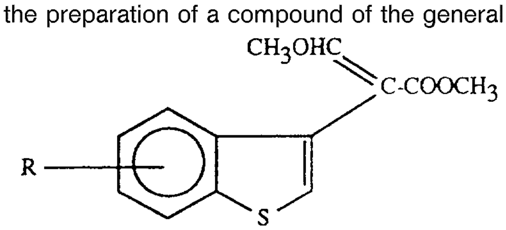

I

in which
R is halogen, $C_{1-4}$alkyl, $C_{1-4}$haloalkyl, $C_{1-4}$alkoxy-$C_{1-4}$alkyl, aryl-$C_{1-4}$alkoxy-$C_{1-4}$alkyl, aryloxy-$C_{1-4}$alkyl, heteroaryloxy-$C_{1-4}$alkyl, arylthio-$C_{1-4}$alkyl, heteroarylthio-$C_{1-4}$alkyl, $C_{2-5}$alkanoyloxy-$C_{1-4}$alkyl, (optionally $C_{1-4}$alkyl-substituted $C_{3-6}$cycloalkyl)-carbonyloxy-$C_{1-4}$alkyl, aroyloxy-$C_{1-4}$alkyl, aryl-$C_{2-5}$alkanoyloxy-$C_{1-4}$alkyl, heteroaryl-$C_{2-5}$alkanoyloxy-$C_{1-4}$alkyl, $C_{1-4}$alkoxy, aryloxy or one of the groups (a) to (e)

$R^1CH=CH$ (a)

$R^2R^3NCH_2$ (b)

$R^4R^5C=NOCH_2$ (c)

$R^6N=CH$ (d)

$R^7R^8N\text{-}N=CH$ (e),

$R^1$ is aryl or heteroaryl,
$R^2$ and $R^3$ independently of one another are hydrogen, $C_{1-4}$alkyl, aryl-$C_{1-4}$alkyl, aryl or heteroaryl,
or
$R^2$ and $R^3$ together with the nitrogen atom to which they are bonded are a 5- or 6-membered ring which may contain an oxygen or sulfur atom,
$R^4$ and $R^5$ independently of one another are hydrogen, $C_{1-4}$alkyl, $C_{1-4}$haloalkyl, $C_{1-4}$alkoxy-$C_{1-4}$alkyl, $C_{1-4}$alkylthio-$C_{1-4}$alkyl, aryl-$C_{1-4}$alkyl,
$C_{3-6}$cycloalkyl, aryl, heteroaryl, $C_{1-4}$alkoxy or aroyl,
or
$R^4$ and $R^5$ together with the carbon atom to which they are bonded are a 5- or 6-membered carbocyclic or heterocyclic ring to which one or two benzene rings may be fused,
$R^6$ is aryl, heteroaryl, $C_{1-4}$alkoxy, aryl-$C_{1-4}$alkoxy or $C_{3\ or\ 4}$alkenyloxy,
and
$R^7$ and $R^8$ independently of one another are hydrogen, $C_{1-4}$alkyl, aryl, heteroaryl or arylsulfonyl, which comprises,

a) to prepare those compounds of the formula I in which R is other than a group (b), (c), (d) or (e), treating a methyl α-(3-benzo[b]thienyl)-β-hydroxyacrylate of the general formula

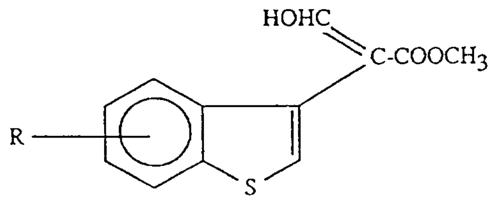

II

in which R is as defined above, with the exception of a group (b), (c), (d) or (e), with a methylating agent,

b) to prepare those compounds of the formula I in which R is bromomethyl, treating a methyl α-(methyl-3-benzo[b]thienyl)-β-methoxyacrylate of the general formula

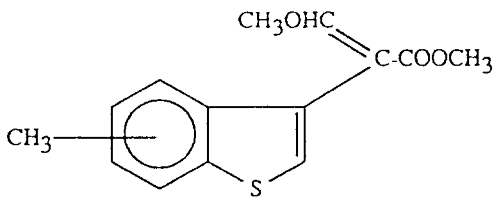

Ia

with N-bromosuccinimide,

c) to prepare those compounds of the formula I in which R is $C_{1-4}$alkoxy-methyl, aryl-$C_{1-4}$alkoxymethyl, aryloxymethyl, heteroaryloxymethyl, arylthiomethyl, heteroarylthiomethyl, $C_{2-5}$alkanoyloxymethyl, (optionally $C_{1-4}$alkyl-substituted $C_{3-6}$cycloalkyl)carbonyloxymethyl, aroyloxymethyl, aryl-$C_{2-5}$alkanoyloxymethyl, heteroaryl-$C_{2-5}$alkanoyloxymethyl, a group (b) or a group (c), reacting a methyl α-(bromomethyl-3-benzo[b]thienyl)-β-methoxyacrylate of the general formula

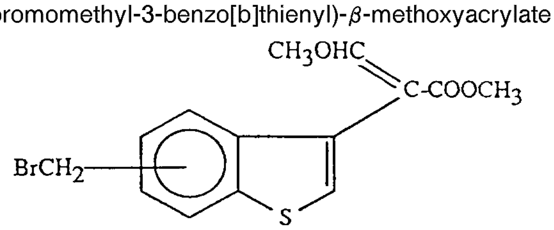

Ib

with a hydroxy compound, a mercapto compound, a carboxylic acid, an amine or an oxime of the general formula

R'-H III

in which R' is $C_{1-4}$alkoxy, aryl-$C_{1-4}$alkoxy, aryloxy, heteroaryloxy, $C_{2-5}$alkanoyloxy, (optionally $C_{1-4}$alkyl-substituted $C_{3-6}$cycloalkyl)-carbonyloxy, aroyloxy, aryl-$C_{2-5}$alkanoyloxy, heteroaryl-$C_{2-5}$alkanoyloxy, arylthio, heteroarylthio, a group $R^2R^3N$ (b') or a group $R^4R^5C=NO$ (c'),

d) to prepare those compounds of the formula I in which R is a group (a), reacting a dialkyl {3-(α-methoxycarbonyl-β-methoxyvinyl)-benzo[b]-thienyl}methylphosphonate of the general formula

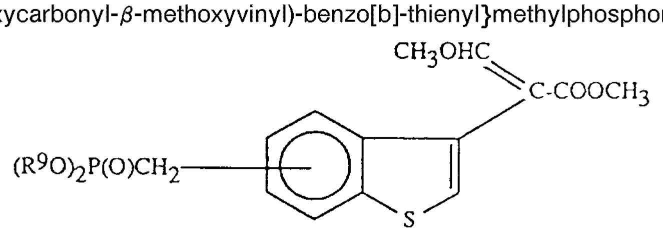

IV

in which $R^9$ is $C_{1-4}$alkyl, with an aldehyde of the general formula

$R^1$CHO V

in which $R^1$ has the abovementioned meaning, or

e) to prepare those compounds of the formula I in which R is a group (d) or a group (e), reacting a methyl $\alpha$-(formyl-3-benzo[b]thienyl)-$\beta$-methoxy-acrylate of the general formula

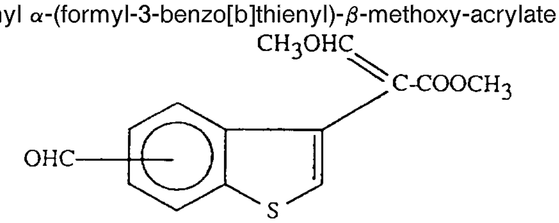

VI

with an amine, hydroxylamine or hydrazine of the general formula

$R^6NH_2$ VII

or

$R^7R^8N\text{-}NH_2$ VIII

in which $R^6$, $R^7$ and $R^8$ are as defined above.

5. The process according to claim 4, which is used for preparing those compounds of the formula I in which R is halogen, $C_{1-4}$alkyl, $C_{1-4}$haloalkyl, $C_{1-4}$alkoxy-$C_{1-4}$alkyl, aryloxy-$C_{1-4}$alkyl, heteroaryloxy-$C_{1-4}$alkyl, arylthio-$C_{1-4}$alkyl, heteroarylthio-$C_{1-4}$alkyl, $C_{1-4}$alkoxy, aryloxy or one of the groups (a) to (d) defined in claim 4, $R^1$, $R^2$ and $R^3$ are as defined in claim 4, and $R^4$ and $R^5$ independently of one another are hydrogen, $C_{1-4}$alkyl, $C_{1-4}$haloalkyl, $C_{1-4}$alkoxy-$C_{1-4}$alkyl, $C_{3-6}$cycloalkyl, aryl or heteroaryl, and $R^6$ is aryl or heteroaryl.

6. A method of controlling fungi in agriculture and in horticulture, which comprises treating the goods to be protected with an effective amount of a composition according to claim 1 or 3.

7. A method of controlling fungi in agriculture and in horticulture, which comprises treating the goods to be protected with an effective amount of a composition according to claim 2.

**Revendications**
**Revendications pour les Etats contractants suivants :AT, BE, CH, DE, FR, GB, IT, LI, LU, NL**

1. Composés de formule générale

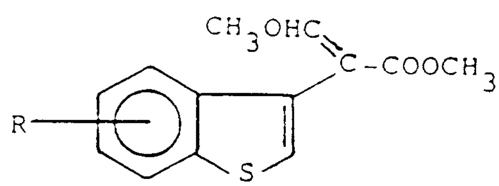

I

dans laquelle

R représente un atome d'halogène ou un groupe alkyle en $C_{1-4}$, halogénoalkyle en $C_{1-4}$, alcoxy($C_{1-4}$)-alkyle($C_{1-4}$), arylalcoxy($C_{1-4}$)-alkyle($C_{1-4}$), aryloxyalkyle($C_{1-4}$), hétéroaryloxyalkyle($C_{1-4}$), arylthioalkyle($C_{1-4}$), hétéroarylthioalkyle($C_{1-4}$), alcanoyloxy-($C_{2-5}$)-alkyle($C_{1-4}$), [cycloalkyl($C_{3-6}$)- éventuellement substitué par un radical alkyle en $C_{1-4}$]-carbonyloxyalkyle($C_{1-4}$), aroyloxyalkyle($C_{1-4}$), arylalcanoyloxy($C_{2-5}$)-alkyle-($C_{1-4}$), hétéroarylalcanoyloxy($C_{2-5}$)-alkyle($C_{1-4}$), alcoxy en $C_{1-4}$, aryloxy ou l'un des groupes (a) à (e)

$R^1CH=CH$ (a)

$R^2R^3NCH_2$ (b)

$R^4R^5C=NOCH_2$ (c)

$R^6N=CH$ (d)

$R^7R^8N-N=CH$ (e),

$R^1$ représente un groupe aryle ou hétéroaryle,

$R^2$ et $R^3$ représentent, indépendamment l'un de l'autre, un atome d'hydrogène ou un groupe alkyle en $C_{1-4}$, arylalkyle($C_{1-4}$), aryle ou hétéroaryle, ou

$R^2$ et $R^3$ forment ensemble, avec l'atome d'azote auquel ils sont liés, un cycle à 5 ou 6 chaînons, contenant éventuellement un atome d'oxygène ou de soufre,

$R^4$ et $R^5$ représentent, indépendamment l'un de l'autre, un atome d'hydrogène ou une groupe alkyle en $C_{1-4}$, halogénoalkyle en $C_{1-4}$, alcoxy($C_{1-4}$)-alkyle($C_{1-4}$), alkylthio-($C_{1-4}$)-alkyle($C_{1-4}$), arylalkyle ($C_{1-4}$), cycloalkyle en $C_{3-6}$, aryle, hétéroaryle, alcoxy en $C_{1-4}$ ou aroyle, ou

$R^4$ et $R^5$ forment ensemble, avec l'atome de carbone auquel ils sont liés, un cycle carbocyclique ou hétérocyclique à 5 ou 6 chaînons, comportant éventuellement un ou deux cycles benzéniques fusionnés,

$R^6$ représente un groupe aryle, hétéroaryle, alcoxy en $C_{1-4}$, arylalcoxy($C_{1-4}$) ou alcényloxy en $C_{3\ ou\ 4}$,

et

$R^7$ et $R^8$ représentent, indépendamment l'un de l'autre, un atome d'hydrogène ou un groupe alkyle en $C_{1-4}$, aryle, hétéroaryle ou arylsulfonyle.

**2.** Composé selon la revendication 1, dans lesquels R représente un atome d'halogène ou un groupe alkyle en $C_{1-4}$, halogénoalkyle en $C_{1-4}$, alcoxy($C_{1-4}$)-alkyle($C_{1-4}$), aryloxyalkyle($C_{1-4}$), hétéroaryloxyalkyle($C_{1-4}$), arylthioalkyle-($C_{1-4}$),hétéroarylthioalkyle($C_{1-4}$), alcoxy en $C_{1-4}$, aryloxy ou l'un des groupes (a) à (d), $R^1$, $R^2$ et $R^3$ ont les significations indiquées dans la revendication 1, et $R^4$ et $R^5$ représentent, indépendamment l'un de l'autre, un atome d'hydrogène ou un groupe alkyle en $C_{1-4}$, halogénoalkyle en $C_{1-4}$, alcoxy($C_{1-4}$)-alkyle($C_{1-4}$), cycloalkyle en $C_{3-6}$, aryle ou hétéroaryle, et $R^6$ représente un groupe aryle ou hétéroaryle.

**3.** Composés selon la revendication 1 ou 2, dans lesquels le substituant R se trouve en position 5 du noyau benzo[b]thiophène.

**4.** Composés selon la revendication 2, choisis parmi les suivants:
α-{5-(α-méthyl-m-trifluorométhyl-benzylidène-amino-oxyméthyl)-3-benzo[b]thiényl}-β-méthoxyacrylate de méthyle,
α-{5-(α-[n-propyl]-benzylidène-amino-oxyméthyl)-3-benzo[b]-thiényl}-β-méthoxyacrylate de méthyle,
α-{5-[α-(2-pyridyl)-éthylidène-amino-oxyméthyl]-3-benzo[b]-thiényl}-β-méthoxyacrylate de méthyle,
α-{5-(α-cyclopropyl-4-chlorobenzylidène-amino-oxyméthyl)-3-benzo[b]thiényl}-β-méthoxyacrylate de méthyle,
α-{5-méthoxy-3-benzo[b]thiényl}-β-méthoxyacrylate de méthyle,
α-{5-éthoxy-3-benzo[b]thiényl}-β-méthoxyacrylate de méthyle,
α-{5-[(α-phényléthyl)méthylaminométhyl]-3-benzo[b]thiényl}-β-méthoxyacrylate de méthyle,
α-{5-(dicyclopropylméthylidène-amino-oxvméthyl)-3-benzo[b]-thiényl}-β-méthoxyacrylate de méthyle,
α-{5-[(cyclopropyl)(2-pyridyl)méthylidène-amino-oxyméthyl]-3-benzo[b]thiényl}-β-méthoxyacrylate de méthyle,
α-(5-(α-cyclopropyl-m-trifluorométhyl-benzylidène-amino-oxyméthyl)-3-benzo[b]thiényl}-β-méthoxyacrylate de méthyle.

**5.** Composés selon la revendication 1, choisis parmi:
l'α-{5-(α-cyclopropyl-benzylidène-amino-oxyméthyl)-3-benzo-[b]thiényl}-β-méthoxyacrylate de méthyle,
l'α-{5-[β-méthyl-α-(2-pyridyl)-propylidène-amino-oxyméthyl]-3-benzo[b]thiényl}-β-méthoxyacrylate de

méthyle et
l'α-{5-(2-isopropyl-4-méthyl-2,5-dihydrothiazol-5-ylidèneamino-oxyméthyl)-3-benzo[b]thiényl}-β-méthoxyacrylate de méthyle.

6. Composition fongicide, caractérisée en ce qu'elle contient une quantité efficace d'au moins un composé de formule générale

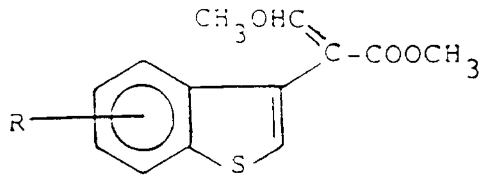

I

dans laquelle

R représente un atome d'halogène ou un groupe alkyle en $C_{1-4}$, halogénoalkyle en $C_{1-4}$, alcoxy($C_{1-4}$)-alkyle($C_{1-4}$), arylalcoxy($C_{1-4}$)-alkyle($C_{1-4}$), aryloxyalkyle($C_{1-4}$), hétéroaryloxyalkyle($C_{1-4}$), arylthioalkyle($C_{1-4}$), hétéroarylthioalkyle($C_{1-4}$), alcanoyloxy-($C_{2-5}$)-alkyle($C_{1-4}$), [cycloalkyl($C_{3-6}$)- éventuellement substitué par un radical alkyle en $C_{1-4}$]-carbonyloxyalkyle($C_{1-4}$), aroyloxyalkyle($C_{1-4}$), arylalcanoyloxy($C_{2-5}$)-alkyle-($C_{1-4}$), hétéroarylalcanoyloxy($C_{2-5}$)-alkyle($C_{1-4}$), alcoxy en $C_{1-4}$, aryloxy ou l'un des groupes (a) à (e)

$R^1CH=CH$ (a)

$R^2R^3NCH_2$ (b)

$R^4R^5C=NOCH_2$ (c)

$R^6N=CH$ (d)

$R^7R^8N\text{-}N=CH$ (e),

$R^1$ représente un groupe aryle ou hétéroaryle,

$R^2$ et $R^3$ représentent, indépendamment l'un de l'autre, un atome d'hydrogène ou un groupe alkyle en $C_{1-4}$, arylalkyle($C_{1-4}$), aryle ou hétéroaryle, ou

$R^2$ et $R^3$ forment ensemble, avec l'atome d'azote auquel ils sont liés, un cycle à 5 ou 6 chaînons, contenant éventuellement un atome d'oxygène ou de soufre,

$R^4$ et $R^5$ représentent, indépendamment l'un de l'autre, un atome d'hydrogène ou une groupe alkyle en $C_{1-4}$, halogénoalkyle en $C_{1-4}$, alcoxy($C_{1-4}$)-alkyle($C_{1-4}$), alkylthio($C_{1-4}$)-alkyle($C_{1-4}$), arylalkyle($C_{1-4}$), cycloalkyle en $C_{3-6}$, aryle, hétéroaryle, alcoxy en $C_{1-4}$ ou aroyle, ou

$R^4$ et $R^5$ forment ensemble, avec l'atome de carbone auquel ils sont liés, un cycle carbocyclique ou hétérocyclique à 5 ou 6 chaînons, comportant éventuellement un ou deux cycles benzéniques fusionnés,

$R^6$ représente un groupe aryle, hétéroaryle, alcoxy en $C_{1-4}$, arylalcoxy($C_{1-4}$) ou alcényloxy en $C_{3\text{ ou }4}$,

et

$R^7$ et $R^8$ représentent, indépendamment l'un de l'autre, un atome d'hydrogène ou un groupe alkyle en $C_{1-4}$, aryle, hétéroaryle ou arylsulfonyle,

ainsi que des adjuvants de formulation.

7. Composition fongicide selon la revendication 6, caractérisée en ce qu'elle contient une quantité efficace d'au moins un composé choisi parmi les suivants:
α-{5-(α-méthyl-m-trifluorométhyl-benzylidène-amino-oxyméthyl)-3-benzo[b]thiényl}-β-méthoxyacrylate de méthyle,

α-{5-(α-[n-propyl]-benzylidène-amino-oxyméthyl)-3-benzo[b]-thiényl}-β-méthoxyacrylate de méthyle,
α-{5-[α-(2-pyridyl)-éthylidène-amino-oxyméthyl]-3-benzo[b]-thiényl}-β-méthoxyacrylate de méthyle,
α-{5-(α-cyclopropyl-4-chlorobenzylidène-amino-oxyméthyl)-3-benzo[b]thiényl}-β-méthoxyacrylate de méthyle,
α-{5-méthoxy-3-benzo[b]thiényl}-β-méthoxyacrylate de méthyle,
α-{5-éthoxy-3-benzo[b]thiényl}-β-méthoxyacrylate de méthyle,
α-{5-[(α-phényléthyl)méthylaminométhyl]-3-benzo[b]thiényl}-β-méthoxyacrylate de méthyle,
α-{5-(dicyclopropylméthylidène-amino-oxyméthyl)-3-benzo[b]-thiényl}-β-méthoxyacrylate de méthyle,
α-{5-[(cyclopropyl)(2-pyridyl)méthylidène-amino-oxyméthyl]-3-benzo[b]thiényl}-β-méthoxyacrylate de méthyle,
α-{5-(α-cyclopropyl-m-trifluorométhyl-benzylidène-amino-oxyméthyl)-3-benzo[b]thiényl}-β-méthoxyacrylate de méthyle,
ainsi que des adjuvants de formulation.

**8.** Composition fongicide selon la revendication 6, caractérisée en ce qu'elle contient une quantité efficace d'au moins un composé choisi parmi:
l'α-{5-(α-cyclopropyl-benzylidène-amino-oxyméthyl)-3-benzo-[b]thiényl}-β-méthoxyacrylate de méthyle,
l'α-{5-[β-méthyl-α-(2-pyridyl)-propylidène-amino-oxyméthyl]-3-benzo[b]thiényl}-β-méthoxyacrylate de méthyle et
l'α-{5-(2-isopropyl-4-méthyl-2,5-dihydrothiazol-5-ylidène-amino-oxyméthyl)-3-benzo[b]thiényl}-β-méthoxyacrylate de méthyle,
ainsi que des adjuvants de formulation.

**9.** Procédé pour la préparation de composés de formule générale

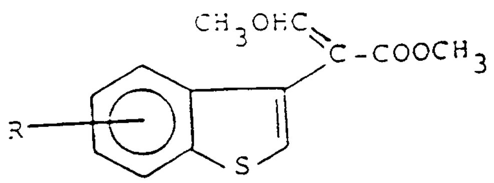

I

dans laquelle

R représente un atome d'halogène ou un groupe alkyle en $C_{1-4}$, halogénoalkyle en $C_{1-4}$, alcoxy($C_{1-4}$)-alkyle($C_{1-4}$), arylalcoxy($C_{1-4}$)-alkyle($C_{1-4}$), aryloxyalkyle($C_{1-4}$), hétéroaryloxyalkyle($C_{1-4}$), arylthioalkyle($C_{1-4}$), hétéroarylthioalkyle($C_{1-4}$),alcanoyloxy-($C_{2-5}$)-alkyle($C_{1-4}$), [cycloalkyl($C_{3-6}$)- éventuellement substitué par un radical alkyle en $C_{1-4}$]-carbonyloxyalkyle($C_{1-4}$), aroyloxyalkyle($C_{1-4}$), arylalcanoyloxy($C_{2-5}$)-alkyle-($C_{1-4}$), hétéroarylalcanoyloxy($C_{2-5}$)-alkyle($C_{1-4}$), alcoxy en $C_{1-4}$, aryloxy ou l'un des groupes (a) à (e)

$R^1CH=CH$ (a)

$R^2R^3NCH_2$ (b)

$R^4R^5C=NOCH_2$ (c)

$R^6N=CH$ (d)

$R^7R^8N-N=CH$ (e),

$R^1$ représente un groupe aryle ou hétéroaryle,

$R^2$ et $R^3$ représentent, indépendamment l'un de l'autre, un atome d'hydrogène ou un groupe alkyle en $C_{1-4}$, arylalkyle($C_{1-4}$), aryle ou hétéroaryle, ou

$R^2$ et $R^3$ forment ensemble, avec l'atome d'azote auquel ils sont liés, un cycle à 5 ou 6 chaînons, contenant éventuellement un atome d'oxygène ou de soufre,

$R^4$ et $R^5$ représentent, indépendamment l'un de l'autre, un atome d'hydrogène ou une groupe alkyle en $C_{1-4}$, halogénoalkyle en $C_{1-4}$, alcoxy($C_{1-4}$)-alkyle($C_{1-4}$), alkylthio-($C_{1-4}$)-alkyle($C_{1-4}$), arylalkyle($C_{1-4}$), cycloalkyle en $C_{3-6}$, aryle, hétéroaryle, alcoxy en $C_{1-4}$ ou aroyle, ou

$R^4$ et $R^5$ forment ensemble, avec l'atome de carbone auquel ils sont liés, un cycle carbocyclique ou hétérocyclique à 5 ou 6 chaînons, comportant éventuellement un ou deux cycles benzéniques fusionnés,

$R^6$ représente un groupe aryle, hétéroaryle, alcoxy en $C_{1-4}$, arylalcoxy($C_{1-4}$) ou alcényloxy en $C_{3\ ou\ 4}$,

et

$R^7$ et $R^8$ représentent, indépendamment l'un de l'autre, un atome d'hydrogène ou un groupe alkyle en $C_{1-4}$, aryle, hétéroaryle ou arylsulfonyle,

caractérisé en ce que

a) pour la préparation des composés de formule I dans lesquels R est différent d'un groupe (b), (c), (d) ou (e), on traite par un agent de méthylation un α-(3-benzo[b]-thiényl)-β-hydroxyacrylate de méthyle de formule générale

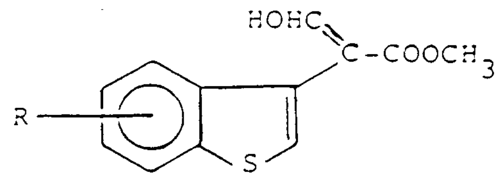

II

dans laquelle R a la signification indiquée plus haut, à l'exception d'un groupe (b), (c), (d) ou (e),

b) pour la préparation des composés de formule I, dans lesquels R représente le groupe bromométhyle, on traite par du N-bromosuccinimide un α-(méthyl-3-benzo[b]thiényl)-β-méthoxyacrylate de méthyle de formule générale

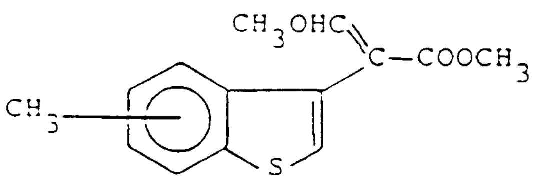

Ia

c) pour la préparation des composés de formule I dans lesquels R représente un groupe alcoxy-($C_{1-4}$)-méthyle, arylalcoxy($C_{1-4}$)-méthyle, aryloxyméthyle, hétéroaryloxyméthyle, arylthiométhyle, hétéroarylthiométhyle, alcanoyloxy($C_{2-5}$)-méthyle, [cycloalkyl($C_{3-6}$)- éventuellement substitué par un groupe alkyle en $C_{1-4}$]-carbonyloxyméthyle, aroyloxyméthyle, arylalcanoyloxy($C_{2-5}$)-méthyle, hétéroaryl-alcanoyloxy($C_{2-5}$)-méthyle, un groupe (b) ou un groupe (c), on fait réagir un α-(bromométhyl-3-benzo[b]thiényl)-β-méthoxyacrylate de méthyle de formule générale

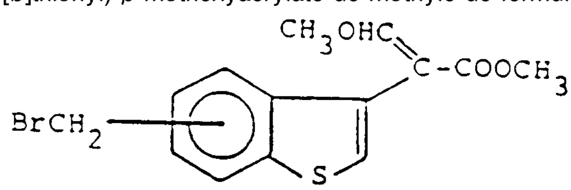

Ib

avec un composé hydroxylé, un composé mercapto, un acide carboxylique, une amine ou une oxime de formule générale

R'-H III

dans laquelle R' représente un groupe alcoxy en $C_{1-4}$, arylalcoxy($C_{1-4}$), aryloxy, hétéroaryloxy, alcanoyloxy en $C_{2-5}$, [cycloalkyl($C_{3-6}$)- éventuellement substitué par un groupe alkyle en $C_{1-4}$]-carbonyloxy, aroyloxy, arylalcanoyloxy($C_{2-5}$), hétéroarylalcanoyloxy($C_{2-5}$), arylthio, hétéroarylthio,

un groupe $R^2R^3N$ (b') ou un groupe $R^4R^5C=NO$ (c'),
d) pour la préparation des composés de formule I dans lesquels R représente un groupe (a), on fait réagir un {3-(α-méthoxycarbonyl-β-méthoxyvinyl)-benzo[b]thiényl}-méthylphosphonate de dialkyle de formule générale

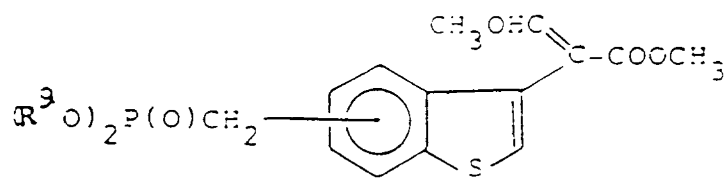

IV

dans laquelle $R^9$ représente un groupe alkyle en $C_{1-4}$, avec un aldéhyde de formule générale

$R^1CHO$ V

dans laquelle $R^1$ a la signification donnée plus haut, ou
e) pour la préparation des composés de formule I dans lesquels R représente un groupe (d) ou un groupe (e), on fait réagir un α-(formyl-3-benzo[b]thiényl)-β-méthoxyacrylate de méthyle de formule générale

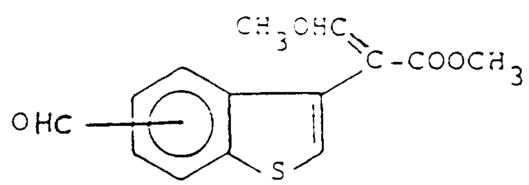

VI

avec une amine, hydroxylamine ou hydrazine de formule générale

$R^6NH_2$ VII

ou

$R^7R^8N\text{-}NH_2$ VIII

formules dans lesquelles $R^6$, $R^7$ et $R^8$ ont les significations données plus haut.

**10.** Procédé selon la revendication 9, caractérisé en ce qu'on l'utilise pour la préparation des composés de formule I dans lesquels R représente un atome d'halogène ou un groupe alkyle en $C_{1-4}$, halogénoalkyle en $C_{1-4}$, alcoxy($C_{1-4}$)-alkyle($C_{1-4}$), aryloxyalkyle($C_{1-4}$), hétéroaryloxyalkyle-($C_{1-4}$),arylthioalkyle-($C_{1-4}$), hétéroarylthioalkyle($C_{1-4}$),alcoxy en $C_{1-4}$, aryloxy ou l'un des groupes (a) à (d) indiqués dans la revendication 9, $R^1$, $R^2$ et $R^3$ ont les significations indiquées dans la revendication 9, et $R^4$ et $R^5$ représentent, indépendamment l'un de l'autre, un atome d'hydrogène ou un groupe alkyle en $C_{1-4}$, halogénoalkyle en $C_{1-4}$, alcoxy($C_{1-4}$)-alkyle($C_{1-4}$), cycloalkyle en $C_{3-6}$, aryle ou hétéroaryle, et $R^6$ représente un groupe aryle ou hétéroaryle.

**11.** Procédé pour la lutte contre des champignons en agriculture et en horticulture, caractérisé en ce que l'on traite l'objet à protéger par une quantité efficace d'un composé selon l'une des revendications 1, 3 et 5 ou d'une composition selon la revendication 6 ou 8.

**12.** Procédé pour la lutte contre des champignons en agriculture et en horticulture, caractérisé en ce que l'on traite l'objet à protéger par une quantité efficace d'un composé selon la revendication 2 ou 4 ou d'une composition selon la revendication 7.

**13.** Utilisation d'un composé selon l'une des revendications 1, 3 et 5 ou d'une composition selon la revendication 6 ou 8, pour la lutte contre des champignons en agriculture et en horticulture.

**14.** Utilisation d'un composé selon la revendication 2 ou 4 ou d'une composition selon la revendication 7, pour la lutte contre des champignons en agriculture et en horticulture.

**Revendications pour l'Etat contractant suivant : ES**

**1.** Composition fongicide, caractérisée en ce qu'elle contient une quantité efficace d'au moins un composé de formule générale

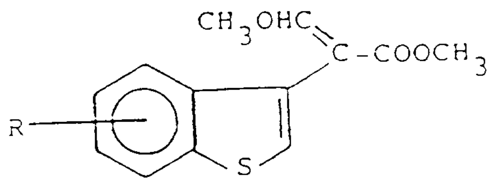

I

dans laquelle

R représente un atome d'halogène ou un groupe alkyle en $C_{1-4}$, halogénoalkyle en $C_{1-4}$, alcoxy($C_{1-4}$)-alkyle($C_{1-4}$), arylalcoxy($C_{1-4}$)-alkyle($C_{1-4}$), aryloxyalkyle($C_{1-4}$), hétéroaryloxyalkyle($C_{1-4}$), arylthioalkyle($C_{1-4}$), hétéroarylthioalkyle($C_{1-4}$), alcanoyloxy-($C_{2-5}$)-alkyle($C_{1-4}$), [cycloalkyl($C_{3-6}$)- éventuellement substitué par un radical alkyle en $C_{1-4}$]-carbonyloxyalkyle($C_{1-4}$), aroyloxyalkyle($C_{1-4}$), arylalcanoyloxy($C_{2-5}$)-alkyle-($C_{1-4}$), hétéroarylalcanoyloxy($C_{2-5}$)-alkyle($C_{1-4}$), alcoxy en $C_{1-4}$, aryloxy ou l'un des groupes (a) à (e)

$R^1CH=CH$ (a)

$R^2R^3NCH_2$ (b)

$R^4R^5C=NOCH_2$ (c)

$R^6N=CH$ (d)

$R^7R^8N\text{-}N=CH$ (e),

$R^1$ représente un groupe aryle ou hétéroaryle,

$R^2$ et $R^3$ représentent, indépendamment l'un de l'autre, un atome d'hydrogène ou un groupe alkyle en $C_{1-4}$, arylalkyle($C_{1-4}$), aryle ou hétéroaryle, ou

$R^2$ et $R^3$ forment ensemble, avec l'atome d'azote auquel ils sont liés, un cycle à 5 ou 6 chaînons, contenant éventuellement un atome d'oxygène ou de soufre,

$R^4$ et $R^5$ représentent, indépendamment l'un de l'autre, un atome d'hydrogène ou une groupe alkyle en $C_{1-4}$, halogénoalkyle en $C_{1-4}$, alcoxy($C_{1-4}$)-alkyle($C_{1-4}$), alkylthio($C_{1-4}$)-alkyle($C_{1-4}$), arylalkyle($C_{1-4}$), cycloalkyle en $C_{3-6}$, aryle, hétéroaryle, alcoxy en $C_{1-4}$ ou aroyle, ou

$R^4$ et $R^5$ forment ensemble, avec l'atome de carbone auquel ils sont liés, un cycle carbocyclique ou hétérocyclique à 5 ou 6 chaînons, comportant éventuellement un ou deux cycles benzéniques fusionnés,

$R^6$ représente un groupe aryle, hétéroaryle, alcoxy en $C_{1-4}$, arylalcoxy($C_{1-4}$) ou alcényloxy en $C_{3\text{ ou }4}$,

et

$R^7$ et $R^8$ représentent, indépendamment l'un de l'autre, un atome d'hydrogène ou un groupe alkyle en $C_{1-4}$, aryle, hétéroaryle ou arylsulfonyle,

ainsi que des adjuvants de formulation.

**2.** Composition fongicide selon la revendication 1, caractérisée en ce qu'elle contient une quantité efficace d'au moins un composé choisi parmi les suivants:
$\alpha$-{5-($\alpha$-méthyl-m-trifluorométhyl-benzylidène-amino-oxyméthyl)-3-benzo[b]thiényl}-$\beta$-méthoxyacrylate

de méthyle,
α-{5-(α-[n-propyl]-benzylidène-amino-oxyméthyl)-3-benzo[b]-thiényl}-β-méthoxyacrylate de méthyle,
α-{5-[α-(2-pyridyl)-éthylidène-amino-oxyméthyl]-3-benzo[b]-thiényl}-β-méthoxyacrylate de méthyle,
α-{5-(α-cyclopropyl-4-chlorobenzylidène-amino-oxyméthyl)-3-benzo[b]thiényl}-β-méthoxyacrylate de méthyle,
α-{5-méthoxy-3-benzo[b]thiényl}-β-méthoxyacrylate de méthyle,
α-{5-éthoxy-3-benzo[b]thiényl}-β-méthoxyacrylate de méthyle,
α-{5-[(α-phényléthyl)méthylaminométhyl]-3-benzo[b]thiényl}-β-méthoxyacrylate de méthyle,
α-{5-(dicyclopropylméthylidène-amino-oxyméthyl)-3-benzo[b]-thiényl}-β-méthoxyacrylate de méthyle,
α-{5-[(cyclopropyl)(2-pyridyl)méthylidène-amino-oxyméthyl]-3-benzo[b]thiényl}-β-méthoxyacrylate de méthyle,
α-{5-(α-cyclopropyl-m-trifluorométhyl-benzylidène-amino-oxyméthyl)-3-benzo[b]thiényl}-β-méthoxyacrylate de méthyle, ainsi que des adjuvants de formulation.

**3.** Composition fongicide selon la revendication 1, caractérisée en ce qu'elle contient une quantité efficace d'au moins un composé choisi parmi:
l'α-{5-(α-cyclopropyl-benzylidène-amino-oxyméthyl)-3-benzo-[b]thiényl}-β-méthoxyacrylate de méthyle,
l'α-{5-[β-méthyl-α-(2-pyridyl)-propylidène-amino-oxyméthyl]-3-benzo[b]thiényl}-β-méthoxyacrylate de méthyle et
l'α-{5-(2-isopropyl-4-méthyl-2,5-dihydrothiazol-5-ylidèneamino-oxyméthyl)-3-benzo[b]thiényl}-β-méthoxyacrylate de méthyle,
ainsi que des adjuvants de formulation.

**4.** Procédé pour la préparation de composés de formule générale

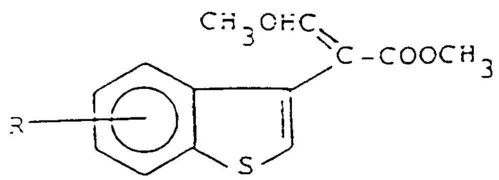

I

dans laquelle

R représente un atome d'halogène ou un groupe alkyle en $C_{1-4}$, halogénoalkyle en $C_{1-4}$, alcoxy($C_{1-4}$)-alkyle($C_{1-4}$), arylalcoxy($C_{1-4}$)-alkyle($C_{1-4}$), aryloxyalkyle($C_{1-4}$), hétéroaryloxyalkyle($C_{1-4}$), arylthioalkyle($C_{1-4}$), hétéroarylthioalkyle($C_{1-4}$), alcanoyloxy-($C_{2-5}$)-alkyle($C_{1-4}$), [cycloalkyl($C_{3-6}$)- éventuellement substitué par un radical alkyle en $C_{1-4}$]-carbonyloxyalkyle($C_{1-4}$), aroyloxyalkyle($C_{1-4}$), arylalcanoyloxy($C_{2-5}$)-alkyle-($C_{1-4}$), hétéroarylalcanoyloxy($C_{2-5}$)-alkyle($C_{1-4}$), alcoxy en $C_{1-4}$, aryloxy ou l'un des groupes (a) à (e)

$R^1CH=CH$ (a)

$R^2R^3NCH_2$ (b)

$R^4R^5C=NOCH_2$ (c)

$R^6N=CH$ (d)

$R^7R^8N\text{-}N=CH$ (e),

$R^1$ représente un groupe aryle ou hétéroaryle,

$R^2$ et $R^3$ représentent, indépendamment l'un de l'autre, un atome d'hydrogène ou un groupe alkyle en $C_{1-4}$, arylalkyle($C_{1-4}$), aryle ou hétéroaryle, ou

$R^2$ et $R^3$ forment ensemble, avec l'atome d'azote auquel ils sont liés, un cycle à 5 ou 6 chaînons, contenant éventuellement un atome d'oxygène ou de soufre,

$R^4$ et $R^5$ représentent, indépendamment l'un de l'autre, un atome d'hydrogène ou une groupe

alkyle en $C_{1-4}$, halogénoalkyle en $C_{1-4}$, alcoxy($C_{1-4}$)-alkyle($C_{1-4}$), alkylthio($C_{1-4}$)-alkyle($C_{1-4}$), arylalkyle($C_{1-4}$), cycloalkyle en $C_{3-6}$, aryle, hétéroaryle, alcoxy en $C_{1-4}$ ou aroyle, ou

$R^4$ et $R^5$ forment ensemble, avec l'atome de carbone auquel ils sont liés, un cycle carbocyclique ou hétérocyclique à 5 ou 6 chaînons, comportant éventuellement un ou deux cycles benzéniques fusionnés,

$R^6$ représente un groupe aryle, hétéroaryle, alcoxy en $C_{1-4}$, arylalcoxy($C_{1-4}$) ou alcényloxy en $C_{3\ ou\ 4}$,

et

$R^7$ et $R^8$ représentent, indépendamment l'un de l'autre, un atome d'hydrogène ou un groupe alkyle en $C_{1-4}$, aryle, hétéroaryle ou arylsulfonyle,

caractérisé en ce que

a) pour la préparation des composés de formule I dans lesquels R est différent d'un groupe (b), (c), (d) ou (e), on traite par un agent de méthylation un α-(3-benzo[b]-thiényl)-β-hydroxyacrylate de méthyle de formule générale

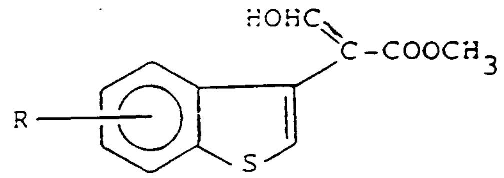

II

dans laquelle R a la signification indiquée plus haut, à l'exception d'un groupe (b), (c), (d) ou (e),

b) pour la préparation des composés de formule I, dans lesquels R représente le groupe bromométhyle, on traite par du N-bromosuccinimide un α-(méthyl-3-benzo[b]thiényl)-β-méthoxyacrylate de méthyle de formule générale

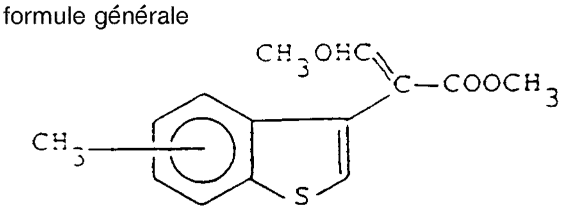

Ia

c) pour la préparation des composés de formule I dans lesquels R représente un groupe alcoxy($C_{1-4}$)-méthyle, arylalcoxy($C_{1-4}$)-méthyle, aryloxyméthyle, hétéroaryloxyméthyle, arylthiométhyle, hétéroarylthiométhyle, alcanoyloxy($C_{2-5}$)-méthyle, [cycloalkyl($C_{3-6}$)- éventuellement substitué par un groupe alkyle en $C_{1-4}$]-carbonyloxyméthyle, aroyloxyméthyle, arylalcanoyloxy($C_{2-5}$)-méthyle, hétéroaryl-alcanoyloxy($C_{2-5}$)-méthyle, un groupe (b) ou un groupe (c), on fait réagir un α-(bromométhyl-3-benzo[b]thiényl)-β-méthoxyacrylate de méthyle de formule générale

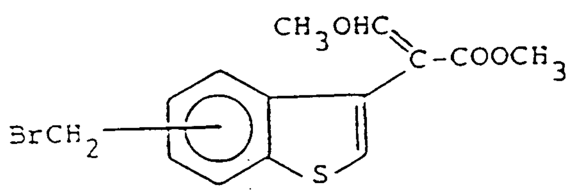

Ib

avec un composé hydroxylé, un composé mercapto, un acide carboxylique, une amine ou une oxime de formule générale

R'-H III

dans laquelle R' représente un groupe alcoxy en $C_{1-4}$, arylalcoxy ($C_{1-4}$), aryloxy, hétéroaryloxy, alcanoyloxy en $C_{2-5}$, [cycloalkyl($C_{3-6}$)- éventuellement substitué par un groupe alkyle en $C_{1-4}$]-carbonyloxy, aroyloxy, arylalcanoyloxy($C_{2-5}$), hétéroarylalcanoyloxy($C_{2-5}$), arylthio, hétéroarylthio, un groupe $R^2R^3N$ (b') ou un groupe $R^4R^5C=NO$ (c'),

d) pour la préparation des composés de formule I dans lesquels R représente un groupe (a), on fait réagir un {3-(α-méthoxycarbonyl-β-méthoxyvinyl)-benzo[b]thiényl}-méthylphosphonate de dialkyle de formule générale

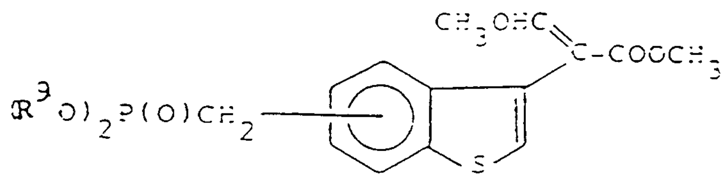

IV

dans laquelle $R^9$ représente un groupe alkyle en $C_{1-4}$, avec un aldéhyde de formule générale

$R^1CHO$ V

dans laquelle $R^1$ a la signification donnée plus haut, ou

e) pour la préparation des composés de formule I dans lesquels R représente un groupe (d) ou un groupe (e), on fait réagir un α-(formyl-3-benzo[b]thiényl)-β-méthoxyacrylate de méthyle de formule générale

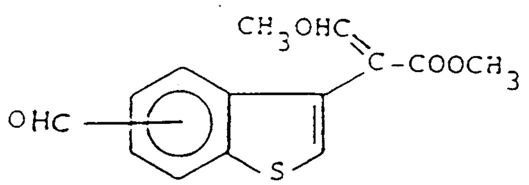

VI

avec une amine, hydroxylamine ou hydrazine de formule générale

$R^6NH_2$ VII

ou

$R^7R^8N\text{-}NH_2$ VIII

formules dans lesquelles $R^6$, $R^7$ et $R^8$ ont les significations données plus haut.

5. Procédé selon la revendication 4, caractérisé en ce qu'on l'utilise pour la préparation des composés de formule I dans lesquels R représente un atome d'halogène ou un groupe alkyle en $C_{1-4}$, halogénoalkyle en $C_{1-4}$, alcoxy($C_{1-4}$)-alkyle($C_{1-4}$ ), aryloxyalkyle($C_{1-4}$), hétéroaryloxyalkyle-($C_{1-4}$),arylthioalkyle-($C_{1-4}$), hétéroarylthioalkyle($C_{1-4}$),alcoxy en $C_{1-4}$, aryloxy ou l'un des groupes (a) à (d) indiqués dans la revendication 4, $R^1$, $R^2$ et $R^3$ ont les significations indiquées dans la revendication 4, et $R^4$ et $R^5$ représentent, indépendamment l'un de l'autre, un atome d'hydrogène ou un groupe alkyle en $C_{1-4}$, halogénoalkyle en $C_{1-4}$, alcoxy($C_{1-4}$)-alkyle($C_{1-4}$), cycloalkyle en $C_{3-6}$, aryle ou hétéroaryle, et $R^6$ représente un groupe aryle ou hétéroaryle.

6. Procédé pour la lutte contre des champignons en agriculture et en horticulture, caractérisé en ce que l'on traite l'objet à protéger par une quantité efficace d'une composition selon la revendication 1 ou 3.

7. Procédé pour la lutte contre des champignons en agriculture et en horticulture, caractérisé en ce que l'on traite l'objet à protéger par une quantité efficace d'une composition selon la revendication 2.